# EUROPEAN PATENT APPLICATION

(11) **EP 4 844 074 A1**
(43) Date of publication of application: **25.11.2026**
(21) Application number: 25462006.5
(22) Date of filing: 22.05.2025
(51) Int. Cl.: C12Q 1/6883

(54) **DETECTING GRAFT INJURY**

(71) Applicant: Immucor GTI Diagnostics Inc., Waukesha, WI 53186 (US)
(72) Inventor: BÉRCES, Attila, 1117 BUDAPEST (HU); BOST, Douglas Andrew, 3543AN UTRECHT (NL); MAK, Cem, 3543AN UTRECHT (NL); BOERRIGTER, Berend Jan, 3543AN UTRECHT (NL)
(74) Representative: ZBM Patents - Zea, Barlocci & Markvardsen

(57) **Abstract**

The present disclosure is directed to a method for detecting donor-derived cell-free nucleic acids in a transplant recipient, said method comprising assaying at least one biological sample derived from the recipient containing cell-free nucleic acids to detect a presence and/or a quantity of one or more markers, wherein the one or more markers consist of one or more donor-informative polymorphisms, and wherein the presence and/or quantity of at least one donor-informative polymorphism is indicative of donor-derived cell-free nucleic acids in the transplant recipient. Also disclosed are methods for determining graft health status in a transplant recipient based on the quantification of donor-derived cell-free nucleic acids. Also disclosed are kits, computer implemented methods and systems for determining donor-derived cell-free nucleic acids and graft health status in a transplant recipient.

## Description

### Technical Field

The present disclosure relates to detecting graft injury in a transplant recipient based on cell-free DNA.

### Background Art

The transplantation of tissues and organs is a critical medical procedure used to replace dysfunctional organ or tissue in a recipient with donor organ or tissue that is genetically or immunologically compatible.

Globally, the demand for organ transplants far exceeds the availability of suitable donors. Unfortunately, some transplants fail due to graft injury. Injury can result from inter alia immunological-based rejection, pharmacological cytotoxicity, trauma, blood flow ischemia and/or infection. Other forms of graft injury, such as viral infection, are also associated with adverse health effects for patients and increased costs associated with treatment.

Reducing transplant failures due to rejection not only increases the availability of donor organs for other patients but also significantly lowers overall healthcare costs. Additionally, it can be highly desirable to detect injury in an early stage before it progresses to organ dysfunction. Such early detection can greatly improve the chances of successful treatment and graft preservation allowing for prompt intervention, for example, with appropriate immunosuppressive treatments, potentially preserving the organ and saving the patient's life.

Currently, the success of a transplant is primarily assessed through an invasive histological examination of biopsied tissue. However, this method has several drawbacks, including a high sampling error rate, low sensitivity, potential graft injury, the need for specialized technical expertise, and high costs.

There is a clear need to improve the detection of graft injury, including graft rejection, particularly in early stages.

### Brief Description of Drawings

Figure 1 shows an example of a computer-implemented method 100 for detecting donor-derived nucleic acids in a transplant recipient.
Figure 2 shows an example system 200 for preparation of one or more reagents targeting at least one donor-informative polymorphism
Figure 3 shows an example system 200 with a robotic pipette 301.
Figure 4 shows an example system 200 with a pickup device 401.
Figure 5 shows an example system 200 with a pushing system 501.
Figure 6 shows an example system 200 with a test plate or rack 201 comprising receptacles 202 and containers 203, a robotic pipette 301, a pickup device 401, a pushing system and an interface 402.
Figure 7 shows an example of a computer system 700 for implementing one or more of any of the CI method steps of the CI methods of the disclosure.
Figure 8 shows digital PCR detection of one CND per detection channel.
Figure 9 shows digital PCR detection of two CNDs per detection channel.
Figure 10 shows digital PCR detection of three CNDs per detection channel.
Figure 11 shows digital PCR detection of four CNDs per detection channel.

### Detailed description

All terms as used in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

The term "graft" refers a biological material comprising cells or tissue, such as, for example, bone marrow, a portion of an organ or a whole organ, that may be transplanted or implanted in or into a subject. In some particular examples, the graft is a whole organ or more than one organ. Examples of organs that can be transplanted or implanted include, but are not limited to, kidney, heart, liver, lung, pancreas, intestine, etc. A portion of an organ may be, for example, a piece of skin, lobe of a liver or lung.

In the sense of the present disclosure the transplanted graft may be an allograft or a xenograft. "Allograft" refers to tissue taken from a genetically non-identical donor of the same species. "Xenograft" refers to tissue taken from another species.

"Cell-free" nucleic acid (or cfNA), as used in herein, refers to nucleic acid found in body fluids such as blood or urine after removing the intact cells, e.g., using methods known in the field such as centrifugation, filtration, phase separation or sedimentation. The health of the graft can be monitored by detecting, e.g., qualitatively or quantitatively measuring, donor specific cell-free nucleic acids in a sample from the recipient. Thus, as used herein, donor-derived nucleic acid within a recipient sample and in relation to graft injury is to be understood as donor-derived cell-free nucleic acids. Cell-free nucleic acid may include both cell-free DNA (cfDNA) and cell-free RNA (cfRNA).

The term "single reaction" or "same reaction" refers to a reaction taking place in one vessel and one moment. In the sense of the present disclosure more than one marker may be detected in a single reaction, meaning that they are detected in the same reaction vessel. Sometimes this is also called "multiplex" detection.

The term "channel" or "detection channel" refers to the set of elements, such as optical filters and dichroic mirrors, that direct light of specific wavelengths to the according fluorescence detectors. Different channels measure differently coloured fluorescent signals (that differ in their wavelengths), such as green, blue or red. A "single channel" measures one fluorescent signal.

A "polymorphism" refers to the presence of two or more variant forms of a specific DNA sequence that can occur among different individuals or populations. The most common type of polymorphism involves variation at a single nucleotide (also called a "single-nucleotide polymorphism", or "SNP"). SNPs include single nucleotide variations, including: substitutions (one nucleotide is substituted by another), nucleotide additions or insertions (one nucleotide is added), and deletions (one nucleotide is deleted). Another type of polymorphisms are copy number variations (CNVs). "Copy number variation" is a phenomenon in which sections of the genome are repeated and the number of repeats in the genome varies between individuals. A CNV is a structural genomic variant that results in confined copy number changes in a specific chromosomal region. CNVs include insertions and deletions as well as more complex changes that involve gains and losses at the same locus. CNV was originally defined as insertions and deletions greater than 1 kb in size but, over time, with the wealth of data emerging from the sequencing of genomes, the operational spectrum of copy number variant (CNVs) has widened to include much smaller events (for example, those >50 base pairs in length). These are distinguished from "single nucleotide polymorphisms (SNPs)" by their genomic size. When the copy number variation is a deletion it is termed as "copy number deletion (CND)".

"Markers", as used herein, refer to biological parameters that aid to determine a particular condition, such as, for example, diagnosing a disease. In the sense of the present disclosure the markers are, in particular, genetic polymorphisms. Thus, throughout the present disclosure "marker", "polymorphism", "marker polymorphism" or "polymorphic marker" are used interchangeably.

"Donor-informative markers" as used herein refers to markers which are distinctive for the donor with respect to the recipient. "Donor-informative polymorphisms" as used herein are thus polymorphisms which are distinctive for the donor with respect to the recipient.

An exemplary donor-informative marker polymorphism in the sense of the present disclosure is a CND comprising from 60 to 100,000 consecutive nucleotides, wherein said nucleotides are present in the genome of the donor but deleted in the genome of the recipient. Otherwise stated, a CND comprising from 60 to 100,000 consecutive nucleotides which are deleted in the genome of the recipient but present (or not deleted) in the genome of the donor. This means that the recipient is nullisomic for the CND and the donor is not.

In other examples, donor-informative marker polymorphism in the sense of the present disclosure is an allele which is present in the genome of the donor but not in the genome of the recipient, said allele comprising at least 5 SNPs, within a DNA polymorphic locus no longer than 120 nucleotides, which are different in the donor and the recipient. This particular allele (containing the combination of at least 5 SNPs within a DNA polymorphic locus no longer than 120 nucleotides) is thus only found in the donor.

To "detect" in the sense of the present disclosure is understood as determining the presence, absence or quantity of an analyte (target). To "quantify" is understood as determining the amount of an analyte (target). In the sense of the present disclosure quantification encompasses semi-quantification and relative quantification. In some embodiments of the disclosure semi-quantitative detection is sufficient, for example, to identify donor-informative markers. In other embodiments, relative quantification is in order, for example, when monitoring the efficacy of a treatment administered to the recipient. In other embodiments, it will be understood that absolute quantification is preferred, for example, for determining if the patient is suffering graft-rejection.

As used herein, the "amount" or "quantity" or "level" may be absolute or relative and may refer to number of copies of a marker detected, concentration of the detected marker, or fraction. The concentration may be expressed as known by the skilled person, typically as nanograms of nucleic acids per ml blood or plasma or copies of markers/ml of blood or plasma (or bodily fluid). In an example, The fraction of donor-derived cfDNA is calculated by dividing the average concentration (or copy number) of donor-informative markers by the concentration (or copy number) of a reference gene used to represent total cfDNA in the sample. Details of such example calculations can be readily understood by one skilled in the art and identified in the field of post transplant monitoring using quantitative methods. Additionally, a relative quantity can be represented as a percentage calculated as a test result quantity such as copy number or concentration of a marker divided by another test result quantity, such as the highest test result quantity, e.g., a copy number or concentration of another marker. In some embodiments, quantities and amounts such as those examples provided herein are used to determine whether or not a quantity or amount is associated with a donor informative marker, and/or additionally providing a determination on graft injury, its existence or severity.

The current disclosure describes methods, kits, assays, components, configurations, systems, and other associated features for *in vitro* testing of a transplant recipient useful to detect donor-derived cell-free nucleic acids in the recipient and determining graft health status.

A first aspect of the present disclosure is directed to an *in vitro* method for detecting donor-derived cell-free nucleic acids in a transplant recipient, said method comprising assaying at least one biological sample derived from the recipient containing cell-free nucleic acids to detect a presence and/or a quantity of one or more markers, wherein the one or more markers consist of one or more donor-informative polymorphisms, and wherein the presence and/or quantity of at least one donor-informative polymorphism is indicative of donor-derived cell-free nucleic acids in the transplant recipient.

A second aspect refers to an *in vitro* method for determining graft health status in a transplant recipient, comprising assaying at least one biological sample derived from the recipient containing cell-free nucleic acids to quantify one or more markers, wherein the one or more markers consist of one or more donor-informative polymorphisms, and outputting information about graft health status based on the quantity of at least one donor-informative polymorphism.

A third aspect refers to a kit or device comprising means for carrying out a method as defined in any one of aspects 1 or 2, said means comprising: (i) a first plurality of containers containing labelled reagents for detecting a plurality of polymorphisms, each container of the first plurality containing labelled reagents directed to more than one polymorphism, and wherein each recipient of the first plurality contains differently labelled reagents directed to each different polymorphism; or (ii) a second plurality of containers containing labelled reagents for detecting the plurality of polymorphisms as defined in (i), each container of the second plurality containing labelled reagents directed to a different polymorphism, wherein the labels are the same or different.

A fourth aspect refers to a computer-implemented method for detecting donor-derived cell-free nucleic acids in a transplant recipient comprising instructing assay performance, the assay comprising assaying at least one biological sample derived from the recipient containing cell-free nucleic acids to detect a presence and/or a quantity of one or more markers, wherein the one or more markers consist of one or more donor-informative polymorphisms. The computer-implemented method further comprises receiving the presence and/or quantity of the one or more donor-informative polymorphisms in the biological sample; and the computer-implemented method further comprises providing result information about donor-derived cell-free nucleic acids in the transplant recipient.

A fifth aspect provides a system for preparing reagents targeting at least one donor-informative polymorphism, the system comprising a test plate or rack comprising a plurality of receptacles, each receptacle of the plurality of receptacles configured to accommodate a container; and wherein the system is configured to receive information relative to a selection of receptacles of the plurality of receptacles wherein the selection of receptacles are configured to accommodate at least a selection of containers, the selection of containers to comprise one or more reagents targeting the at least one donor-informative polymorphism.

A further aspect refers to an in vitro method for detecting donor-derived cell-free nucleic acids in a transplant recipient, said method comprising assaying at least one biological sample derived from the recipient containing cell-free nucleic acids to detect the presence and/or quantity of two or more donor-informative polymorphisms in a single reaction using labelled reagents directed at said donor-informative polymorphisms, wherein all labelled reagents contain the same detectable label, and wherein detecting at least one donor-informative polymorphism is indicative of donor-derived cell-free nucleic acids in the transplant recipient. For example, two or more donor-informative polymorphisms are detected using a single channel, while a single channel or multiple channels can be used in the single reaction.

The disclosed methods, kits, assays, components, configurations, systems and other associated features can provide advantages. For example, they can detect donor-derived cell-free nucleic acids in the recipient even when said donor-derived nucleic acids are present in very low amounts, which may allow detection of graft injury, such as graft rejection or viral infection, at a very early stage.

This donor-derived DNA may be released in very small amounts from apoptotic cells during normal life-cycle of the graft or in greater amounts when the graft is suffering injury. Injury can result from inter alia immunological-based rejection, pharmacological cytotoxicity, trauma, blood flow ischemia and/or infection. The status of the graft can be determined by monitoring cell-free donor-derived nucleic acids in the recipient, and early signs of graft injury identified may lead to appropriate clinical intervention to save the tissue. However, in practice, the amount of donor-derived cell-free DNA in the transplant recipient is so small that it often borders the detection limit of even the most sensitive techniques, such as digital PCR. This is particularly relevant for non-rejecting grafts or in early rejection.

In some situations, as in kidney transplants, a sample from a non-rejecting recipient can have as little as 4 to 5 genome equivalents of donor-derived cell-free DNA in 1 ml of plasma. This means that each monosomic marker is represented by as little as 4 to 5 detectable fragments in the sample derived from 1 ml of plasma. In a standard blood extraction what provides 10 ml whole blood, from which 4 ml of plasma are obtained, there are no more than about 16 to 20 detectable fragments per marker. Moreover, the available sample may need to be further divided, e.g., aliquoted, depending on the number of markers to be detected and typically at least 10-fold, in order to detect multiple markers. This results in further dilution of the measurable markers, leaving about 1-2 measurable molecules of each marker per aliquot, which is close to the detection limit of digital PCR.

The methods disclosed herein comprise specifically detecting the presence and/or quantity of markers which have been previously identified as being donor-informative. The donor-informative markers can be detected cumulatively in a single reaction vessel using one or more detection channels, without the need to differentiate the detection of each individual marker. This is because all markers are informative, and accordingly, there is no need to differentiate individual markers. Measuring only donor-informative markers in a single reaction significantly increases the analytical performance of the method because no sample is wasted to test non-informative markers and the measurable molecules can be increased by a factor of the number of markers in the assay. For this reason, the present methods allow for detecting and quantifying very low amount of target molecules, as in the case of targeting donor-derived cell-free nucleic acids present in a transplant recipient.

While being able to detect and quantify low amounts of donor-derived cell-free nucleic acids in the recipient with improved sensitivity, the present methods, kits, assays, components, configurations, systems, and other associated features provide relevant biomedical information about the status of the graft, which in turn is of great value to the healthcare professional, who may choose the most appropriate treatment regimen for the patient accordingly.

Altogether, the present methods, kits, assays, components, configurations, systems and other associated features provide the following advantages: improved limit of detection for donor-derived cell-free nucleic acids, improved coefficient of variation (especially at low graft fraction), decreased hands-on time, and decreased cost. Additional advantages are related to the potential to assess multiple sample types such as urine and plasma for kidney recipients, or bronchoalveolar lavage (BAL) and plasma for lung recipients, allowing to make differential diagnosis by comparing the results of urinary vs. plasma or BAL vs plasma donor-derived cell-free nucleic acids levels or their changes in a time series. Comparison of results from different samples can provide additional information about comprehensive diagnosis or prognosis of graft health.

Further, all methods of the present disclosure are applicable to allografts as well as xenografts, i.e., they can be used in allotransplants as well as xenotransplants.

### The methods

The methods according to the first and second aspects comprise assaying at least one biological sample derived from the recipient containing cell-free nucleic acids to detect a presence and/or a quantity of one or more markers, wherein the one or more markers consist of one or more donor-informative polymorphisms.

Typically, the donor-informative polymorphism in the sense of the present disclosure may be selected from: (i) a copy number deletion (CND) comprising from 60 to 100,000 consecutive nucleotides which are present in the genome of the donor but deleted in the genome of the recipient, or (ii) an allele present in the genome of the donor but not in the genome of the recipient, said allele comprising at least 5 SNPs, within a DNA polymorphic locus no longer than 120 nucleotides, which are different in the donor and the recipient, in particular wherein said DNA polymorphic locus is within a HLA region. In embodiments, a plurality of donor-informative polymorphisms may be used as markers, including combinations of CNDs as defined in (i), alleles containing SNPs as defined in (ii), or any combinations thereof.

It is important that the donor-informative polymorphisms (for example, the CNDs or the allele as defined above) have been previously identified as those distinctive for the donor with respect to the recipient, in particular, those present in the genome of the donor but not in the genome of the recipient. Thus, in embodiments, the methods of the present disclosure further comprise a prior step of identifying the donor-informative polymorphisms or, alternatively, of receiving the information about the donor-informative polymorphisms. In particular embodiments, the donor-informative polymorphisms are present in the germline genome of the donor but not in the germline genome of the recipient.

Targeting solely donor-informative polymorphic markers provides the advantage of drastically reducing, even eliminating, background noise in the assay: since the marker is present in the donor but not in the recipient, a signal detected by targeting the marker will necessarily come from the donor. Furthermore, in embodiments with a plurality of donor-informative polymorphisms used as markers, since all markers are informative, there is no need to differentiate individual markers and, consequently, the cumulative amount of all donor-informative markers can be detected in a single reaction vessel, using the same or different detection channels. Altogether the result is a significant increase in sensitivity, analytical performance and convenience. While quantification of the donor-derived cell-free nucleic acids is not mandatory to conclude that donor-derived nucleic acids are present in the recipient, the improved performance achieved with the present method allows for precise quantification. Quantification of donor-derived cell-free nucleic acids enables providing additional and improved information related to the health status of the graft.

In a preferred embodiment, the quantity of donor-derived cell-free nucleic acids is detected.

In embodiments, the donor-informative marker polymorphisms for the methods of the disclosure are CNDs. In embodiments, the CNDs are deletions of 20-100,000 consecutive nucleotides, in particular of 60 to 100,000 consecutive nucleotides, in particular of 60-500 consecutive nucleotides, more particularly of 200-500, for example, about 200, about 220, about 240, about 260, about 280, about 300, about 320, about 340, about 360, about 380, about 400, about 420, about 440, about 460, about 480, or about 500 consecutive nucleotides. By "deletions of x-y consecutive nucleotides" it is meant that the deleted sequence consists of x to y consecutive nucleotides. Using CNDs as the donor-informative polymorphic markers has the advantage of eliminating background noise, further improving the performance of the methods.

In other embodiments, a useful donor-informative marker polymorphism in the sense of the present disclosure comprises an allele present in the genome of the donor but not in the genome of the recipient, said allele comprising at least 5 SNPs which are different in the donor and the recipient within a DNA polymorphic locus no longer than 120 nucleotides. It is to be understood that each marker allele comprises at least five single nucleotide SNPs which may be at any location within the DNA polymorphic locus. The DNA polymorphic locus is one that may have sufficient genomic diversity to distinguish individuals within an ethnic group or population. In particular, the DNA polymorphic locus is in the leukocyte antigen (HLA) region of the genome. More in particular, DNA polymorphic locus is in the major histocompatibility complex (MHC) region of the genome, for example, the locus comprising the at least 5 SNPs is within an intron of MHC. In particular embodiments, the useful marker allele comprises 6 or more, 7 or more, 8 or more, 9 or more, or 10 or more single nucleotide SNPs.

A variety of analytical techniques for performing the assay may be used. In particular, an analytical technique is selected that is sufficiently sensitive to identify presence of or to quantify the CNDs or allele as defined above. In some embodiments, the assay is performed by a quantitative technique, such as one selected from the group consisting of digital PCR (dPCR), quantitative PCR (q-PCR), single cell PCR, restriction fragment length PCR (PCR-RFLP), PCR-RFLP/RT-PCR-RFLP^ hot start PCR, nested PCR, in situ polonony PCR, in situ rolling circle amplification, bridge PCR, picotiter PCR or emulsion PCR. Other amplification methods include selective amplification of target polynucleotide sequences, consensus sequence primed PCR (CP-PCR), arbitrarily primed PCR (AP-PCR), degenerate oligonucleotide-primer PCR (DOP-PCR), nucleic acid-based sequence amplification (NABSA), and sequencing. In a particular example, dPCR may be used, including droplet digital PCR (ddPCR). dPCR, as well as the other techniques mentioned above, is well known in the field, and several providers market equipment and reagents for dPCR. Exemplary commercial platforms for performing dPCR are Qiacuity from Qiagen, BioRad QX600, Digital QuantStudio, and Roche Digital LightCycler.

The advantage of using a quantitative technique is that the quantity of donor-derived cell-free nucleic acids that may be detected in the recipient is correlated to the extent of graft injury, i.e. by quantifying the amount of copies or genomic equivalents of donor-informative markers/polymorphisms in the sample derived from the recipient it is possible to determine the extent of graft injury. For example, as will be explained below, a fraction of donor-derived cell-free nucleic acids above 0.5% can be associated to risk of allograft injury in kidney transplant recipients.

In embodiments, the assay comprises cumulatively detecting the presence and/or quantity, preferably the quantity, of more than one donor-informative marker polymorphism. For example, from 2 to 20 donor-informative polymorphisms may be typically quantified. In particular, from 4 to 16 donor-informative polymorphisms, for example, 4, 5, 6, 7, 8, 9, 10, 11, 12, 13, 14, 15, or 16 donor-informative polymorphisms are quantified. In particular embodiments, the donor-informative polymorphisms are detected cumulatively in a single reaction. This means that all donor-informative marker polymorphisms are determined in the same reaction vessel. As mentioned above, this is possible because all informative marker polymorphisms are informative (are only present in the donor, not in the recipient). However, the donor-informative polymorphisms may also be detected in more than one reaction, for example, groups of 4 to 8 donor-informative markers may be determined, each group in a different reaction vessel or container. As will be detailed below, several donor-informative marker polymorphisms can be detected cumulatively in the same detection channel, because there is no need to differentiate donor-informative markers. Alternatively, different donor-informative marker polymorphisms can be detected in different detection channels.

In embodiments, the assay is carried out using labelled reagents directed to the donor-informative marker polymorphisms. In particular embodiments, the reagents comprise a plurality of oligonucleotide sequences or complements thereof directed at each one of the donor-informative polymorphisms. Typically, the reagents comprise nucleotide primers and probes. By "directed at" it is meant that the reagents are designed to specifically bind to at least part of the target donor-informative polymorphisms. This means that the reagents target a specific donor-informative polymorphism. In the present disclosure "directed at" and "targeting" are used interchangeably. "Specifically bind" in the context of the present disclosure preferably means that the reagents hybridize to the target genomic sequence containing said donor-informative polymorphisms.

Typically, the reagents comprise a compound that emits a detectable signal, also called a "label". Both primers and probes may be labelled, although, typically, only the probes are labelled. The label is typically a compound that emits a detectable signal, such as, for example, a fluorophore, a colorimetric compound, a radioisotope. In one example the labels are fluorescent molecules.

As mentioned above, the assay does not require that the different donor-informative markers are individually detected, such that multiple donor-informative polymorphisms may be quantified cumulatively in a single reaction in each detection channel. This means that the reagents directed at the different donor-informative markers may all be labelled with the same fluorophore. However, it may be convenient in some circumstances to have different labels for targeting different donor-informative polymorphic markers.

The reagents typically comprise primers configured to amplify the target region of the genome, i.e. the region containing the CND or the at least 5 SNPs, as well as labelled probes that are configured to bind to the amplicons and emit a detectable signal which can be used to detect/quantify the target donor-informative marker polymorphism.

In one example, the reagents comprise primers configured to amplify a region within the donor-informative CND polymorphism. In particular examples, the primers are configured to amplify a region typically no longer than 120 nucleotides within a CND. This is advantageous for detecting donor-derived cell-free DNA because cell-free DNA tends to be fragmented. In embodiments, the primers are designed to amplify a region around 100, or around 60 nucleotides long within the CND. Since designed to amplify a region that is deleted in the recipient, target amplification will only take place if donor-derived nucleic acids are present in the sample. Moreover, the labelled probes are typically complementary to a nucleic acid fragment within the amplicon, such that a signal will only be emitted if donor-derived cell-free nucleic acids are present in the sample. When using a quantitative technique such as dPCR the amount of donor-derived cell-free nucleic acids can be detected. Instead, when the probe does not find its target nucleic acid fragment (because it is deleted and has not been amplified), then it cannot bind and there will be no signal, meaning that no donor-derived cell-free nucleic acids are present in the sample.

When the donor-informative polymorphic marker is an allele comprising at least 5 SNPs as defined above, the reagents typically comprise primers designed to amplify the region containing the at least 5 SNPs and a probe designed to bind a region within the amplicon. The length of the region to be amplified is preferably as short as possible. In this case, the SNPs may be in the primer binding site or the probe binding site, such that, theoretically, the primer or the probe do not bind in the absence of the 5 SNPs, and no signal is detected. However, in practice, since the binding is thermodynamic, some residual unspecific binding may take place even in the absence of the 5 SNPs, resulting in a residual signal. Consequently, in this case, the absence of donor-derived cell-free nucleic acids may be associated to no signal or to a residual (very low) signal that is close to 0. On the other side, when donor-derived cell-free nucleic acids are present in the sample, both the primers and the probe will bind their targets and a consistent signal will be emitted. Again, when a quantitative technique such as dPCR is used the amount of donor-derived cell-free nucleic acids can be detected. The skilled person will be able to distinguish between a residual baseline signal associated to a negative result (absence of donor-derived cell-free nucleic acids) and the significantly higher signal associated to a positive result (presence of donor-derived cell-free nucleic acids).

The reagents for the assay may also contain primers and labelled probes directed at a control sequence, also called "housekeeping" sequence or gene.

In particular examples, the reagents comprise fluorescent hydrolysis probes (commonly known as "TaqMan probes"). These probes contain a fluorescent label and a quencher. While both fluorescent label and quencher are attached to the probe, the fluorescence of the dye is suppressed by the quencher. The probe is designed to specifically bind to a target sequence. During amplification, the probe is cleaved by the DNA polymerase, resulting in the separation of the fluorophore and quencher, leading to a fluorescent signal. This signal is directly proportional to the amount of target nucleic acid present in the sample.

The biological sample(s) derived from the recipient comprise cell-free nucleic acids. For example, the biological sample(s) may be a body fluid, such as one selected from blood, plasma, urine, bronchoalveolar lavage (BAL), serum, pus, respiratory fluid, lymph fluid, feces, bile, saliva, sputum, semen, vaginal flow, cerebrospinal fluid, brain fluid, ascites, milk, secretions from the genitourinary tract, and combinations thereof. In some embodiments, the biological sample is selected from plasma, urine, or bronchoalveolar lavage. In particular, the sample may be plasma or comprise plasma. However, sometimes it is useful to use other type of biological fluids as samples, in particular, urine and BAL. It may be advantageous to use multiple results of the measurements from different fluids. For example, in particular examples, the donor-derived cell-free nucleic acids may be measured in plasma and urine, or plasma and BAL samples. Performing the assay in different samples may provide advantages. For example, some particular conditions may be detected by comparing prevalence of donor cfDNA in recipient urine samples as compared to recipient plasma samples.

Preferably, the cell-free nucleic acids are extracted from the sample(s) and used for performing the assay. Extracting cell-free nucleic acids from a biological sample can be carried out by methods well known to the skilled person, for example, it may be done with commercial extraction kits following manufacturer's instructions.

The donor-informative marker polymorphisms for use in the assay described above may be identified in a prior assay or they may be simply provided. In some embodiments, the donor-informative marker polymorphisms are identified by receiving and analysing existing genotyping data of the donor and the recipient and identifying the donor-informative polymorphisms as those that are present in the genome of the donor and deleted or not found in the genome of the recipient. In other embodiments, the donor-informative polymorphisms are identified by genotyping of the donor and the recipient and identifying the donor-informative polymorphisms as those that are present in the genome of the donor and deleted or not found in the genome of the recipient. Genotyping is well known to the skilled person and analysis of the resulting information may also be carried out by well-known bioinformatic methods. For example, genotyping performed on the donor and/or recipient earlier, e.g., in preparation for a transplant, may be used to establish the donor-informative polymorphisms. Genotyping of the donor may be performed at a different time from genotyping of the recipient.

In embodiments, donor-informative CNDs are identified by (a) performing a prior assay on at least one biological sample derived from the recipient containing cell-free nucleic acids to detect a plurality of CNDs, and (b) identifying the donor-informative CNDs as a subset of the plurality of CNDs which are detected to be present in the genome of the donor but deleted in the genome of the recipient. Determining CNDs enables a highly sensitive approach of identifying non-recipient nucleic acids. Another advantage is that the detection of CNDs does not require sequence analysis or bi-allelic microchimerism analysis. In embodiments, the CNDs are deletions of 20-100,000 consecutive nucleotides, in particular of 60 to 100,000 consecutive nucleotides, in particular of 60-500 consecutive nucleotides, more particularly of 200-500, for example, about 200, about 220, about 240, about 260, about 280, about 300, about 320, about 340, about 360, about 380, about 400, about 420, about 440, about 460, about 480, or about 500 consecutive nucleotides.

In embodiments, for example, when the graft is an allograft, each of the CNDs in the plurality of CNDs determined in the prior assay are characterised by being found in a frequency ranging from 40 to 60% of a population. If the prevalence of the CND in the target population is either very low or very high, then it is more likely that both donor and recipient will have the same variant and, thus, it will not be possible to differentiate the source of the CND as being from the donor or from the recipient or from both. In embodiments, each of the CNDs of the plurality for carrying out the prior assay are found in a frequency of around 50% of a target population. A skilled person will be familiar with methods for CND selection and frequency calculations, such as, for example, the methods disclosed in WO2013049892, the entire content of which is incorporated herein by reference.

Table 1 provides an example of a plurality of CND sites useful in the practice of the present methods. However, other suitable CNDs can be selected as for carrying out a prior assay in which to identify donor-informative CNDs. The CNDs in Table 1 are referred to as "CND 0X" where X is a numeral from 1 to 10. The list in Table 1 should be read in conjunction with, and includes, other known CNDs such as those defined in Conrad et al. (2010); McCarroll et al. (2008).

**Table 1**

| ***Copy Number Deletion Polymorphisms*** | | | | | | | | | |
|---|---|---|---|---|---|---|---|---|---|
| **CND ID** | **CNP_id** | **chr** | **start hg18** | **end hg18** | **SIZE bp** | **0 Copy, frequency** | **1 Copy frequency** | **2 Copy frequency** | **CNV Info** |
| **CND_01** | CNVR376.1 | chr1 | 157.134,152 | 157,136.674 | 2,522 | 0.508 | 0.418 | 0.074 | agenic |
| **CND_02** | CNVR7344.1 | chr18 | 53,097,735 | 53,099,702 | 1.967 | 0.508 | 0.393 | 0.082 | agenic |
| **CND_03** | CNVR4014.1 | chr8 | 112,363,260 | 112,365,469 | 2,209 | 0.500 | 0.410 | 0.082 | agenic |
| **CND_04** | CNVR6084.1 | chr14 | 21,951,540 | 21,952,070 | 530 | 0.451 | 0.418 | 0.107 | Intronic |
| **CND_05** | CNVR6676.1 | chr16 | 25,247,611 | 25,250,595 | 2.984 | 0.443 | 0.459 | 0.074 | agenic |
| **CND_06** | CNVR3319.1 | chr7 | 24,004,755 | 24,006,584 | 1,829 | 0.434 | 0.344 | 0.139 | agenic |
| **CND_07** | CNVR5850.1 | chr13 | 37,955.318 | 37,958,191 | 2,873 | 0.426 | 0.434 | 0.139 | agenic |
| **CND_08** | CNVR3753 full | chr8 | 4,110,308 | 4,112,335 | 2,027 | 0.426 | 0.426 | 0.131 | Intronic |
| **CND_09** | CNVR7301.1 | chr18 | 33,560,073 | 33,560,645 | 572 | 0.418 | 0.451 | 0.123 | agenic |
| **CND_10** | CNVR4886.1 | chr10 | 108,020,303 | 108,022,518 | 2,215 | 0.410 | 0.418 | 0.172 | agenic |

Table 2 discloses other polymorphisms that can be used in the prior assay.

*Table 2: GRCh37*/*hg19 genome coordinates of 41 CNV loci identified for prior assay development.*

| CNV # | CNV ID | Chromosome | Start | End | Size (bp) |
|---|---|---|---|---|---|
| 1 | CNVR376.1 | chr1 | 158867528 | 158870050 | 2522 |
| 2 | CNVR7344.1 | chr18 | 54946737 | 54948704 | 1967 |
| 3 | CNVR4014.1 | chr8 | 112294084 | 112296293 | 2209 |
| 4 | CNVR6084.1 | chr14 | 22881700 | 22882230 | 530 |
| 5 | CNVR6676.1 | chr16 | 25340110 | 25343094 | 2984 |
| 6 | CNVR3319.1 | chr7 | 24038230 | 24040059 | 1829 |
| 7 | CNVR5850.1 | chr13 | 39057318 | 39060191 | 2873 |
| 8 | CNVR3753_full | chr8 | 4122900 | 4124927 | 2027 |
| 9 | CNVR7301.1 | chr18 | 35306075 | 35306647 | 572 |
| 10 | CNVR4886.1 | chr10 | 108030313 | 108032528 | 2215 |
| 11 | CNVR1138.3 | chr2 | 220052107 | 220054328 | 2221 |
| 12 | CNVR3451.1 | chr7 | 73829106 | 73831236 | 2130 |
| 13 | CNVR4596.1 | chr10 | 4708559 | 4710493 | 1934 |
| 14 | CNVR801_full | chr2 | 54565628 | 54567472 | 1844 |
| 15 | CNVR5853_full | chr13 | 39933627 | 39935387 | 1760 |
| 16 | CNVR842.1 | chr2 | 76773726 | 76775435 | 1709 |
| 17 | CNVR431.1 | chr1 | 187464835 | 187466483 | 1648 |
| 18 | CNVR4203.1 | chr9 | 17910038 | 17911633 | 1595 |
| 19 | CNVR952.1 | chr2 | 121797526 | 121798787 | 1261 |
| 20 | CNVR1037.1 | chr2 | 159959794 | 159960963 | 1169 |
| 21 | CNVR1138.2 | chr2 | 220053192 | 220054328 | 1136 |
| 22 | CNVR5923.1 | chr13 | 72845782 | 72846891 | 1109 |
| 23 | CNVR8147.1 | chr22 | 35645445 | 35646472 | 1027 |
| 24 | CNVR3004.1 | chr6 | 95193393 | 95194392 | 999 |
| 25 | 1343* | chr8 | 112294279 | 112295260 | 981 |
| 26 | CNVR381.1 | chr1 | 159648762 | 159649629 | 867 |
| 27 | CNVR4374.1 | chr9 | 89154952 | 89155776 | 824 |
| 28 | CNVR2799.1 | chr6 | 18402120 | 18402881 | 761 |
| 29 | CNVR6540.1 | chr15 | 99574727 | 99575462 | 735 |
| 30 | CNVR6357.1 | chr15 | 39744398 | 39744844 | 446 |
| 31 | 158 | chr1 | 210081613 | 210083984 | 2371 |
| 32 | CNVR358.1 | chr1 | 152555610 | 152590091 | 34481 |
| 33 | CNVR217.1 | chr1 | 72766282 | 72811969 | 45687 |
| 34 | CNVR7808.1 | chr20 | 21286267 | 21288529 | 2262 |
| 35 | 933 | chr6 | 32431552 | 32573771 | 142219 |
| 36 | 554 | chr4 | 10214156 | 10235268 | 21112 |
| 37 | CNVR2469.1 | chr5 | 57323526 | 57333742 | 10216 |
| 38 | CNVR3009.1 | chr6 | 100034554 | 100035273 | 719 |
| 39 | CNVR3609.1 | chr7 | 148073126 | 148076330 | 3204 |
| 40 | 801 | chr5 | 57326027 | 57333533 | 7506 |
| 41 | CNVR2304.1 | chr5 | 1924604 | 1925111 | 507 |

In embodiments, for example, when the graft is a xenograft, each of the CNDs in the plurality of CNDs determined in the prior assay is characterised by being a sequence that is not found in humans and present in the (non-human) donor.

The prior assay may be carried out using a quantitative or semiquantitative technique. In embodiments, the prior assay is performed with an analytical technique that is sufficiently sensitive to identify presence of or to quantify the CNDs. In some embodiments, the assay is performed by a quantitative technique, such as one of those defined above. In embodiments, dPCR may be used for the prior assay using reagents as described above. All examples and embodiments defined above for the analytical technique also apply to the prior assay.

In embodiments, the plurality of CND markers in the prior assay contains from 10 to 100 markers, in particular from 20 to 60 markers, more particularly from 30 to 60 markers, for example, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 markers. In embodiments, the prior assay comprises quantifying more than one different CND in a single reaction using differently labelled reagents directed at each different CND. Ideally, as many markers as possible are determined in a single reaction. However, multiplex assays have a limit of targets, such that, in practice, it is not possible to detect so many markers in a single reaction. This is because, typically, the markers are identified by a detectable signal, such a fluorescent signal, and most detection platforms have a limited amount of detection channels to simultaneously detect different signal types, e.g., different fluorescent signals. Most platforms have 4 to 6 detection channels, which conditions that only 4 to 6 target markers can be detected in a single reaction. In one example, from 2 to 8 CNDs are detected in a single reaction, in particular from 3 to 6, for example 4, 5, or 6 CNDs are detected in a single reaction. Usually, a control will also be detected in each reaction to inform whether the testing is functioning properly.

In one exemplary lay-out, the prior assay is configured to detect a total of 48 CNDs. Each 4 CNDs are grouped for one single reaction using reagents, typically primers and labelled probes, each set of primers and labelled probes directed at one of the 4 CNDs, wherein each probe directed at a different CND is labelled with a distinctive fluorescent colour from the other 3. Typically, a control target (also known as house-keeping gene) is also detect in each single reaction using reagents containing yet a different label. All embodiments described above for the reagents also apply to the prior-assay.

In one example, a sample obtained from a recipient is aliquoted into 12 sample aliquots, each for detecting a set of 4 different CNDs and a control. The 12 aliquots may typically derive from aliquoting cell-free nucleic acid derived from a plasma sample obtained from a standard blood extraction.

Identifying the donor-informative CNDs comprises detecting the CDNs of the plurality that are present in the genome of the donor but deleted in the genome of the recipient. The informative CNDs to be selected in the prior assay are those for which the CND marker is found in an amount or level below a certain reference value or within a range of reference values that indicates that it is donor-derived. In general, methods for obtaining reference values are available to the skilled person. The reference can be an absolute value, a relative value, a value that has an upper or a lower limit, a range of values, an average value, a median value, a mean value, or a value as compared to a particular control or baseline value.

In the absence of rejection, cell-free nucleic acids from the donor are typically 200 to 500-fold lower than cell-free nucleic acids coming from the recipient. Since the amount of donor-derived cell-free nucleic acids is far lower than that coming from the recipient, the skilled person may discern which signals are coming from donor-derived nucleic acids, for example by using a quantitative or semiquantitative technique and may also establish appropriate reference values. In a relevant example, the reference value may be established by comparing the different levels of CND abundance in a sample taken from the recipient, where the CNDs detected in highest quantities represent the recipient-derived CNDs (including double positives) and CDNs present in relatively lower quantities, e.g., 10 to 500 times lower than the highest quantity, represent those which are only donor-derived (and deleted in the recipient). The reference value or range may be expressed as a % of the most abundant CNDs detected in the assay. In some embodiments, a CND which is present in less than 10%, 5%, or 2% or lower of the most abundant CNDs detected in the assay may be associated with only donor cell-free DNA. However, the skilled person will be aware that the percentage of CND corresponding with a conclusion that the CND is present in the donor genome but deleted in the recipient genome may vary depending upon the organ or tissue of the graft. As an example, a presence of the CND up to less than 1%, 2%, 5%, or 10% or more of the most abundant CNDs detected in the assay may be associated with only donor-derived cell-free DNA in kidney, heart, and lung recipients, whereas a prevalence of the CND up to 20% of the most abundant CNDs detected in the assay may be associated with only donor-derived cell-free DNA in liver recipients. In another example, a non-rejecting recipient can have around 4 to 5 genome equivalents of donor-derived cell-free DNA in 1 ml of plasma. This means that each one-copy marker is represented by as little as 4 to 5 detectable fragments in the 1 ml of plasma. The amount of donor-derived cell-free nucleic acids can be distinguished from recipient nucleic acids, which will have around 5 to 50 detectable fragments in the 1 ml of plasma.

A typical prior-assay may comprise performing qPCR for quantifying a plurality of CNDs (selected as described above). For each target CND one of the following results may be obtained in the prior-assay:
(i) no target CND, meaning that said CND is deleted both in the donor and in the recipient; or
(ii) a first quantity of the target DNA, said first quantity being indicative of the CND being present (not deleted) in the recipient; or
(iii) a second quantity of the target DNA, said second quantity being below a reference value or within a range of reference values which is indicative of the CND being deleted in the recipient but present (not deleted) in the donor, and
b) identifying the target CND as donor-informative when the quantity detected for said CND is the second quantity.

In the sense of the present disclosure, a result consistent with no target CND (i) encompasses detecting zero quantity for the CND, but also detecting a baseline quantity of CND that can be categorized as noise.

As already mentioned above, the skilled person will be aware that amount of cell-free nucleic acid coming from the recipient vs that coming solely from the donor may vary depending upon the organ or tissue of the graft. For example, donor-derived cell-free nucleic acid may be higher in liver transplant recipients as in kidney, lung, or heart transplant recipients. Thus, in one example, when considering a recipient that has received a kidney, a heart or a lung, the second quantity corresponding with a conclusion that the CND is present in the donor but deleted in the recipient may be below 20%, 10% or 5%, 2%, 1%, or 0.2% or less of the most abundant CNDs detected in the assay. However, in another example, when considering a recipient that has received a liver, the second quantity corresponding with a conclusion that the CND is present in the donor but deleted in the recipient may be below 20% of the most abundant CNDs detected in the assay. Typically, the second quantity is at least 10-500 or more times lower than the first quantity.

In some examples, the first and second quantities can both be number of copies, or concentration of number of copies per volume of sample, or the fraction defeined herein. In some embodiments, the comparison of multiple quantities from the test results allows identifications of the first and second quantities, and the zero or baseline quanty. In a situation where such initial quantities used are number of copies, concentrations and fractions can be readily derived or calculated based on the quantities in (i) or (ii) above.

The biological sample(s) derived from the recipient for the prior assay comprise cell-free nucleic acids. For example, the biological sample(s) may be a body fluid as defined above. All examples and embodiments defined above for the biological samples also apply to the prior assay.

When the donor-informative polymorphisms are SNPs, these are typically identified by performing prior analysis of multiple genome data to detect highly polymorphic loci of DNA not longer than 120 nucleotide bases which contain at least five SNPs. In embodiments, the at least five SNPs are identified by genotyping, either by performing a prior genotyping assay or by receiving and analysing prior existing genotyping information.

The second aspect comprises outputting information about graft health status based on the quantity of at least one donor-informative polymorphism. Effectively, the second aspect contemplates quantifying donor-derived cell-free nucleic acids in a transplant recipient as defined above and outputting information about graft health status based on this amount.

In a relevant example, the absence of cell-free donor-derived nucleic acids or a low level of cell-free donor nucleic acids relative to a reference is indicative of no or minimal injury of the graft, meaning that the graft is healthy, and no rejection or infection is taking place. On the other side, the presence of cell-free donor nucleic acids relative to a reference, *i.e.,* exceeding a threshold as measured in absolute terms or as a percentage, is indicative of ongoing injury of the graft. Moreover, increasing amounts of donor-derived cell-free nucleic acids over time are positively related to increasing severity of graft injury, such that the status of the graft can be monitored.

The levels of donor-derived cell-free nucleic acids that are considered to be "normal" or "acceptable" and are indicative of a healthy graft will be apparent to the skilled person, and may vary for different types of grafts and different injuries. Examples of cut-off values for normal or acceptable are described in Whitlam et al (American Journal of Transplantation, Volume 19, Issue 4, 1037 - 1049). The skilled person is also capable to establish the levels of cell-free nucleic acids that are considered to be indicative of graft injury. Thus, the skilled person can easily establish a reference relevant to ongoing injury of the graft. In general terms, this reference can be an absolute value, a relative value, a value that has an upper or a lower limit, a range of values, an average value, a median value, a mean value, or a value as compared to a particular control or baseline value. The reference can be based on an individual sample or from a large number of samples, such as from a population of subjects of a chronological age-matched group or based on a pool of samples including or excluding the sample to be tested. The skilled person in the art, making use of the general knowledge, can choose the subject or group of subjects more adequate for obtaining the reference for each of the methods of the present disclosure. Methods for obtaining the reference from the group of subjects selected are well-known in the state of the art, for example, as disclosed in Burtis et al. (Burtis et al., 2008, Chapter 14, section "Statistical Treatment of Reference Values"). It is noted that the references for determining graft health status may be different from those to identify donor-informative polymorphisms, because the presence of donor-derived cell-free nucleic acids is not necessarily indicative of graft damage. There is usually a baseline amount of donor-derived cell-free nucleic acids in a transplant recipient. However, when the amount of donor-derived cell-free nucleic acids increases, then graft damage can be suspected and, when the amount rises above a certain reference, then graft injury can be stated. For example, the reference for making a diagnosis of graft injury can be established as the amount of donor-derived cell-free nucleic acids circulating in a recipient or group of recipients which do not suffer from graft injury. This reference can be a value or a range of values corresponding to a control recipient or group of control recipients for which no graft injury has been established. For example, a concentration of donor-derived cell-free nucleic acids above 0.5% fraction can already be associated to some risk of allograft injury in certain organ recipients, such as kidney recipients. In other examples, 1-5%, or 2-5%, or 3.5%-5% or above may represent severe graft injuries in certain organ recipients, such as kidney recipients. As fraction is a well-known defined quantity in the field, the thresholds for different types of graft injuries are known in the field as well. Levels of donor-derived cell-free nucleic acids in different clinical scenarios and subpopulations of kidney rejection have also been described, for example, by Aubert et al., Nat. Med. 30, 2320-2327 (2024)(10.1038/s41591-024-03087-3).

In other circumstances, for example, when monitoring graft injury or determining the efficacy of a therapy, the reference may be an amount of donor-derived cell-free nucleic acids circulating in the same recipient being tested but at an earlier timepoint. In an embodiment, a baseline may be established by testing the patient at a time the graft is believed to show no signs of rejection or minimal signs of rejection.

Quantifying donor-derived cell-free nucleic acids can provide additional output information besides information about graft status. Said outputted information may comprise at least one or more of monitoring graft health, screening for graft rejection, risk of suffering from graft rejection, monitoring of graft rejection, determining the efficacy of a therapy for graft rejection, diagnosis of graft rejection, prognosis of graft rejection, diagnosis of viral infections, prognosis of viral infections, monitoring of viral infections, determining the efficacy of a therapy for viral infections, suggestions on additional testing such as biopsy, or a combination thereof.

Each of the outputs mentioned above may give rise to a particular method, for example, when the output relates to graft health status, it is provided a method for determining the health status of a graft comprising detecting donor-derived cell-free nucleic acids as defined above, preferably detecting their quantity, and outputting information about graft status based said detection. In this method, the absence of cell-free donor nucleic acids or a level of cell-free donor nucleic acids relative to a reference is indicative of the absence of ongoing injury. In one example, the level of donor-derived nucleic acids below a reference value or within a range of reference values is indicative of graft injury. In particular, the level of donor-derived cell-free nucleic acids above a reference value or within a range of reference values is indicative of graft injury.

In one example, the quantity of donor-derived cell-free nucleic acids is positively correlated to the severity of graft injury, such that monitoring of health graft status is possible with the method. As such, the method can be disclosed as a method for monitoring changes in the health status of a graft comprising determining the amount of donor-derived cell-free nucleic acids as defined above and outputting information about changes in the health status of the graft based on said determination. In this method, increased levels of donor-derived cell-free nucleic acids relative to a control or relative to the level of donor-derived cell-free nucleic acids determined for the same recipient in an earlier time-point is indicative of a worsening in the health status of the graft. Furthermore, in transplant recipients experiencing variable clinical signs of rejection, the donor-derived cell-free nucleic acid levels may vary, which correlates with the immunologic mechanism of rejection (i.e. humoral versus cellular) as well as the medical category of rejection (i.e. acute versus chronic).

Also provided herein are methods to detect or diagnose graft rejection comprising determining the amount of donor-derived cell-free nucleic acids as defined above and outputting information about the detection or diagnosis of graft rejection based on said determination. In this method, the absence of donor-derived cell-free nucleic acids or a level of donor-derived cell-free nucleic acids below or within a control is indicative of absence of graft rejection. Alternatively, the presence of donor-derived cell-free nucleic acids or a level of donor-derived cell-free nucleic acids above or within a control is indicative of absence of graft rejection. In one relevant example, the method is for early diagnosis or early detection of graft rejection.

Also provided herein is a method to determine the risk of suffering from graft rejection comprising determining the amount of donor-derived cell-free nucleic acids as defined above and outputting information about the risk of suffering from graft rejection based on said determination. For example, increasing levels of donor-derived cell-free nucleic acids may be indicative of increasing risk of graft rejection.

As outlined above, the information provided by the present methods about the status of the graft may lead to appropriate clinical intervention to save the graft. The efficacy of the administered therapy or medical regime may also be monitored by using the methods described herein. Thus, also provided herein is a method to determine or monitor the efficacy of a therapy for graft rejection comprising determining the amount of donor-derived cell-free nucleic acids as defined above and outputting information on the efficacy of the therapy for graft rejection based on determination. In this method, when the level donor-derived cell-free nucleic acids is lower than that determined before the start of the therapy or at an earlier phase of the therapy, this is indicative of efficacy.

Also provided herein is a method for recommending or administering an appropriate medical regime (therapy) for a transplant recipient, comprising determining the amount of donor-derived cell-free nucleic acids as defined above and recommending or administering an appropriate medical regime based on said determination of donor-derived cell-free nucleic acids. In this method, an appropriate medical regime or therapy is recommended or administered when a level of donor-derived cell-free nucleic acids is determined that is indicative of graft injury.

In all the above methods, the determination of donor-derived cell-free nucleic acids can be semi-quantitative or quantitative, preferably quantitative.

The medical regime in the above methods may comprise administering immunosuppressive drugs or, if the patient is on immunosuppressive drugs, a change in dose of the drug or the type of drug. Other medical regimes include anti-pathogen therapy, anti-infection therapy, drugs to improve blood flow or protocols to reduce swelling or trauma to the tissue, such as anti-inflammatory drugs. In other examples the medical regime comprises surgery, for example a surgical intervention for removing the graft.

### Kits or devices

Also provided herein is a kit or device comprising means for carrying out any of the methods described above. For example, the means comprise reagents for determining one or more polymorphic markers. In particular examples, the kit or device further comprises containers containing the reagents inside. In one example, the kit or device further comprises a solid support. In particular examples the solid support contains a plurality of receptacles. Non-limiting examples of appropriate solid supports are well plates or racks. The containers may be placed in the receptacles of the solid support. Appropriate containers are, for example, test tubes, such as, for example, Eppendorf tubes, tubes with screw caps, etc. Alternatively, the reagents can be directly contained in the receptacles of the solid support (for example, inside the wells of a well plate). In one example, the kit or device comprises a plurality of reagents targeting polymorphic markers inside a plurality of containers. In other examples, the kit or device comprises a plurality of reagents targeting polymorphic markers directly distributed inside a plurality of receptacles in the solid support.

In particular examples, each container or receptacle contains reagents targeting a plurality of polymorphic markers. For example, each container or receptacle may contain a plurality of reagents targeting at least 2, or at least 3, or at least 4, or at least 5, or at least 6, markers. This configuration of the kit is appropriate for carrying out the prior assay, where a plurality of polymorphic markers are determined to find out donor-informative markers. Preferably, the targeted polymorphic markers are a plurality of CNDs. In examples, each container or receptacle additionally contains reagents targeting a control sequence (for example, a housekeeping gene). Preferably, each container or receptacle contains labelled reagents directed to more than one polymorphism, preferably more than one CND, wherein each container or receptacle contains differently labelled reagents directed to each different polymorphism. All features described above for said plurality of polymorphic markers of the prior assay also apply to the kit or device.

In other particular examples, each container or receptacle contains reagents targeting only one polymorphic marker. In particular, the kit or device may comprise as many containers containing reagents as the number of targeted polymorphic markers. For example, the kit or device may comprise as many containers containing reagents as the number of targeted polymorphic markers, preferably CNDs, configuring the plurality of markers of the prior assay. This configuration of the kit or device is appropriate for preparing the assay to determine donor-informative markers (the prior assay as described above); once the donor-informative polymorphic markers, preferably CNDs, are identified, the reagents targeting said donor-informative markers may be selected from the plurality of containers or receptacles and used in a single reaction assay. In this case, the labels in the reagents may be the same or different, since the individual markers need not be individually determined. Preferably, the kit or device in this case further comprises at least a further container or receptacle, said further container or receptacle containing reagents targeting a control sequence (for example, a housekeeping gene), which may also be added to the single reaction.

In other embodiments the kit or device comprises reagents, typically labelled reagents, targeting only donor-informative markers. This configuration is appropriate for determining the presence or quantity of donor-informative markers when said donor-informative markers have been already identified. The polymorphic markers in these embodiments may be selected from CNDs or alleles present in the genome of the donor but not in the genome of the recipient, said alleles comprising at least 5 SNPs which are different in the donor and the recipient within a DNA polymorphic locus no longer than 120 nucleotides. The reagents targeting the donor-informative markers may be contained in a single container or receptacle, or in more than one container or receptacle. In particular examples, the reagents targeting the donor-informative markers are contained in a single container or receptacle, which is configured to carry out a single reaction. The labels in the reagents may be the same or different, since the individual donor-informative markers need not be individually determined.

In a particular example, the kit or device comprises: (i) a first plurality of containers or receptacles containing labelled reagents for determining a plurality of polymorphic markers, each container or receptacle of the first plurality containing labelled reagents directed to more than one polymorphic marker, for example 4 different polymorphisms, and wherein each container or receptacle of the first plurality contains differently labelled reagents directed to each different polymorphic marker; and (ii) a second plurality of containers or receptacles containing labelled reagents for determining the plurality of polymorphic markers as defined in (i), each container or receptacle of the second plurality containing labelled reagents directed to a different polymorphism, wherein the labels are the same or different. The polymorphic markers in these embodiments are preferably CNDs. The polymorphic markers in (ii) are donor-informative, i.e. are present in the donor and deleted in the recipient.

The reagents in the kits or devices typically comprise a plurality of oligonucleotide sequences or complements thereof directed at the polymorphic markers, or to a control sequence (such as a housekeeping gene). The reagents, in particular primers and labelled probes, have been disclosed in more detail above. All examples and embodiments described above for the reagents also apply to the kits or devices.

In particular example, the elements of the kits or devices are compatible with digital PCR equipment.

Also provided herein is using the kits or devices as described above in any of the methods of the present disclosure. This includes using the kits or devices for determining graft health status in a transplant recipient, for diagnosing or detecting graft rejection, for the prognosis of graft rejection, for determining the risk of suffering from graft rejection, for monitoring of graft rejection, for determining the efficacy of a therapy for graft rejection, or for recommending or administering a medical regime for graft rejection.

### Computer implemented methods

Also provided herein is a computer-implemented method for detecting donor-derived cell-free nucleic acids in a transplant recipient as defined in the fourth aspect.

Typically, the donor-informative polymorphisms in the fourth aspect are selected from: (i) a copy number deletion (CND) which is present in the genome of the donor but deleted in the genome of the recipient, or (ii) an allele present in the genome of the donor but not in the genome of the recipient, said allele comprising at least 5 SNPs which are different in the donor and the recipient within a DNA polymorphic locus no longer than 120 nucleotides. All embodiments described above for the donor-informative polymorphisms of the assay also apply to the computer implemented methods.

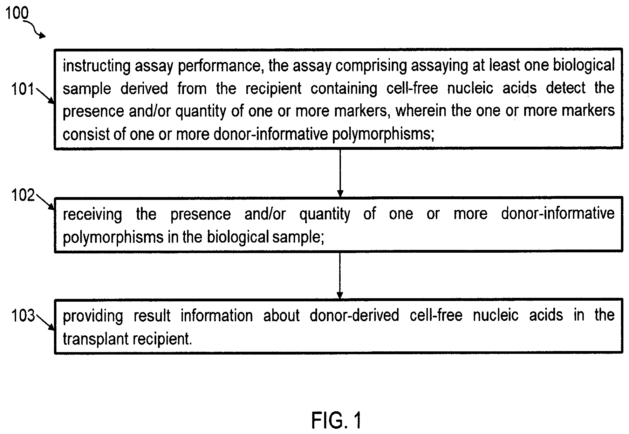

In examples, and with reference to figure 1, a computer-implemented method, CI method, 100 for detecting donor-derived cell-free nucleic acids in a transplant recipient comprises, as seen in block 101, instructing assay performance, the assay comprising assaying at least one biological sample derived from the recipient containing cell-free nucleic acids to detect the presence and/or quantity of one or more markers, wherein the one or more markers consist of one or more donor-informative polymorphisms. The computer-implemented method, CI method, 100 comprises, as seen in block 102, receiving the presence and/or quantity of the one or more donor-informative polymorphisms in the biological sample; CI method, 100 comprises, as seen in block 103, providing result information about donor-derived cell-free nucleic acids in the transplant recipient. The different steps 101, 102, 103 may be performed by the same software or different software in communication with each other. The same software and/or the different software may reside on the same hardware or different hardware connected through a network.

In examples, the CI method comprises instructing assay performance to a system performing the assay, for example, to a thermocycler or to a digital PCR system. In examples of the CI method, instructing the assay performance comprises instructing a thermocycler or a digital PCR system to perform PCR, preferably dPCR, on the biological sample using the reagents targeting the one or more donor-informative polymorphisms. In examples, instructing the thermocycler to perform PCR comprises instructing the thermocycler or to a digital PCR system to perform cycles of heating and cooling appropriate for amplifying the donor-informative polymorphic sequences (CNDs or the alleles comprising SNPs as defined above).

In examples, the CI method comprises receiving the presence and/or quantity of one or more donor-informative polymorphisms from a system performing the assay, for example, a thermocycler or a digital PCR system.

In examples, the CI method comprises providing result information about donor-derived cell-free nucleic acids in the transplant recipient to a user interface, e.g., a graphical interface. The result information may be raw data from the assay, processed data from assay, or clinical support information, such as warnings on graft injury or deterioration of the graft health.

In examples, the CI method may comprise receiving information about, e.g., identification of, the donor-informative polymorphisms (e.g. CNDs or alleles comprising SNPs as defined above). In examples, the information about the donor-informative polymorphisms (e.g. CNDs or alleles comprising SNPs as defined above) are received from a prior assay, from a database, from analysing existing genotyping data of the donor and the recipient, or from genotyping.

In examples, the CI method may comprise instructing a prior assay performance on a biological sample containing cell-free nucleic acids obtained from the recipient to determine a first plurality of polymorphisms. In preferred examples, the plurality of polymorphisms in the prior assay comprises CNDs. All examples and embodiments described above for the CNDs of the prior assay also apply here.

In examples, the CI method may further comprise identifying donor-informative polymorphisms (e.g. CNDs) as a subset of the plurality of polymorphisms (e.g. CNDs) which are present in the genome of the donor and deleted in the genome of the recipient in the prior assay.

In examples of the CI method, identifying the donor-informative polymorphisms (e.g. CNDs) is based on a detectable signal indicating the presence of donor-informative polymorphisms, the detectable signal resulting from the prior assay. In examples, the selection is based on the detectable signal indicating that the polymorphism is present in the donor and deleted in the recipient. The detectable signal resulting from the prior assay may be correlated to an amount of the (one or more) target polymorphism, which, in turn indicates that said nucleic acid is present in the sample and is donor-derived. The amount of CND or range of CND amounts that indicate that said nucleic acid sequence is donor-derived can be determined by the skilled person without undue burden. In examples, the computer-implemented, CI, method comprises determining that the CND(s) belong to the donor based on the one or more concentrations of CND(s). In examples, the computer-implemented method comprises comparing the one or more concentrations of CND with respective to one or more reference values or range of values to determine whether the one or more CND(s) belong to the donor. Instructions and examples about the reference values for determining whether CNDs in the prior assay are donor-derived are described above.

In examples, the CI method may further comprise selecting the donor-informative polymorphisms (e.g. CNDs or alleles comprising SNPs as defined above) to generate or prepare one or more reagents targeting the donor-informative polymorphism for the recipient.

The CI method may further comprise communicating with or controlling hardware to automatically obtain one or more reagents targeting the donor-informative polymorphism comprising a plurality of oligonucleotide sequences or complements thereof directed at the donor-informative polymorphisms (e.g. CNDs or alleles comprising SNPs as defined above).

In examples of the CI method, instructing the prior assay performance comprises instructing a digital PCR system or a thermocycler to perform PCR on a biological sample. In examples, instructing the PCR system to perform PCR comprises instructing the PCR system to perform cycles of heating and cooling appropriate for amplifying the polymorphisms, e.g., the CNDs or the alleles comprising SNPs as defined above. Typically, the PCR is dPCR.

In examples, the CI method comprises sending information relative to a selection of receptacles of a plurality of receptacles comprising containers including one or more reagents targeting the donor-informative polymorphism, to a system as defined in the disclosure. In examples, sending information comprises sending information relative to a selection of receptacles which are correlated with the plurality of polymorphisms, e.g., CNDs or alleles comprising SNPs as defined above, of the one or more reagents targeting the donor-informative polymorphisms. In examples, the information relative to the selection of receptacles is based on a table identifying the positions of a test plate or rack 201, as defined in the disclosure, occupied by containers including the one or more reagents targeting the donor-informative polymorphisms, e.g., CNDs or alleles comprising SNPs as defined above.

In examples, the CI method further comprises outputting information about the selection of receptacles via a user interface, e.g., a graphical interface.

In examples, the CI method further comprises determining the presence and/or quantity of donor-derived cell-free nucleic acids in the recipient.

In examples, the CI method further comprises determining and/or providing or outputting result information about a graft health status based on the determination of the presence and/or quantity of donor-derived cell-free nucleic acids in the recipient, e.g., on a user interface, or on a graphical interface.

In examples, the CI method further comprises determining and/or providing or outputting information about a diagnosis of graft rejection based on the determination of the presence and/or quantity of donor-derived cell-free nucleic acids in the recipient.

In examples, the CI method further comprises determining and/or providing or outputting information about a prognosis of graft rejection based on the determination of the presence and/or quantity of donor-derived cell-free nucleic acids in the recipient.

In examples, the CI method further comprises determining and/or providing or outputting information about a risk of suffering of graft rejection based on the determination of the presence and/or quantity of donor-derived cell-free nucleic acids in the recipient.

In examples, the CI method further comprises determining and/or providing or outputting information about a monitoring of graft health status based on the determination of the presence and/or quantity of donor-derived cell-free nucleic acids in the recipient.

In examples, the CI method further comprises determining and/or providing or outputting information about a efficacy of a therapy for graft rejection based on the determination of the presence and/or quantity of donor-derived cell-free nucleic acids in the recipient.

In examples, the CI method further comprises determining and/or providing or outputting information about an appropriate medical regime for the recipient based on the determination of the presence and/or quantity of donor-derived cell-free nucleic acids in the recipient.

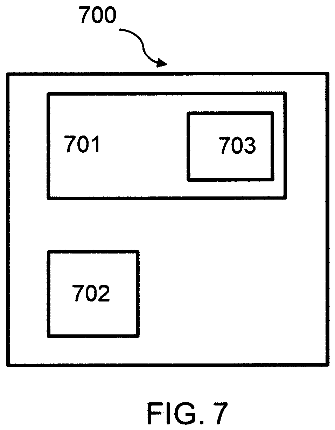

In examples of the disclosure one or more computer systems may be provided which may be each configured to implement one or more of any of the CI method steps of the CI methods of the disclosure. The one or more computer systems may be connected to each other and to any of the systems described in the present disclosure either locally or over an external network, e.g., over the internet. In the disclosure, various functions performed by the one or more computer systems are described as being performed by certain components or modules. Figure 7 shows an example of a computer system 700. However, any division of components and modules is for illustration only. In examples, a function described as performed by a particular component or module may be divided amongst multiple components or modules. Moreover, in alternate examples, two or more components or modules may be integrated into a single component or module. Each component or module may be implemented using hardware (e.g., a field-programmable gate array (FPGA) device, an application-specific integrated circuit (ASIC), a digital signal processor (DSP), a controller, etc.), software (e.g., instructions executable by a processor), or any combination thereof. Any one or more of the computing systems 700 may include a memory 701, one or more processors 702, or a combination thereof. The memory 701 includes a computer-readable medium (e.g., a computer-readable storage device) that stores instructions 703 that are executable by processor 702. The instructions 703 are executable to initiate, perform or control operations to implement any one or more of the steps of the CI method of the disclosure. The processor 702 can be implemented as a single processor or as multiple processors, such as in a multi-core configuration, a multi-processor configuration, a distributed computing configuration, a cloud computing configuration, or any combination thereof.

### Systems

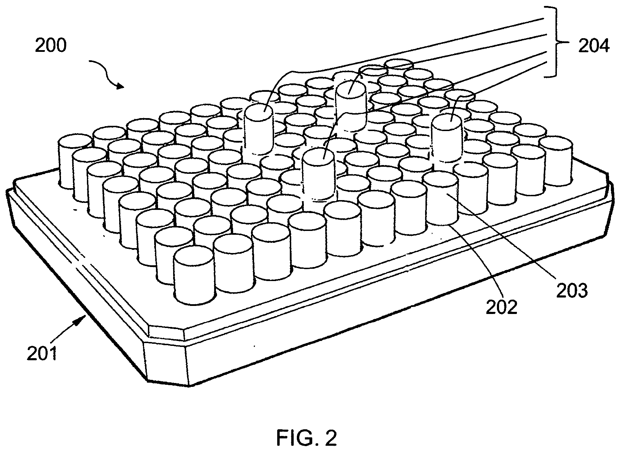

In some examples, the disclosure provides, with reference to figure 2, an example system 200 for preparation of one or more reagents targeting one or more donor-informative polymorphisms, the system comprising a test plate or rack 201 comprising a plurality of receptacles 202, each receptacle of the plurality of receptacles configured to accommodate a container 203; and wherein the system 200 is configured to receive information relative to a selection of receptacles of the plurality of receptacles wherein the selection of receptacles are configured to accommodate at least a selection 204 of containers, the selection of containers to comprise one or more reagents targeting the one or more donor-informative polymorphisms.

In examples, the one or more donor-informative polymorphism is identified as described in examples above.

In examples the reagents comprise primers and labelled probes targeting the one or more donor-informative polymorphisms. The reagents have been described above. All examples and embodiments about the reagents described above also apply to the system.

In examples the system is further configured to
- obtain or receive at least the one or more reagents targeting the one or more donor-informative polymorphisms, for example the system comprising and using one or more of a reagents input, feeder, loading station, loader, cartridge feeder, cassette, supply module, input tray, input module,
   and
- to prepare the one or more reagents into one or more containers, for example the system comprising and using one or more of: robotic arms, pipettes or a combination thereof to, e.g., insert or add one or more reagents into one or more containers.

In examples, each receptacle of the plurality of receptacles 202 accommodates a container 203. In examples each container 203 contains reagents directed to a single polymorphism of the plurality of polymorphisms as defined above for the prior assay.

In examples, the system further comprises at least the selection 204 of containers.

The test plate or rack 201 may comprise rows, e.g., labelled as 1...8, and may comprise columns e.g., labelled as A...L, of receptacles in a matrix, where each position may be labeled by its position in the respective row and columns, for example, receptacle 5B. The information relative to the selection of receptacles comprising the selection 204 of containers with the one or more reagents targeting the donor-informative polymorphism may allow the system to indicate to a robotic arm or to a user which containers, e.g., test tubes, are to be picked for performing the assay of the methods of the disclosure, for example, selection 204 of containers or test tubes 4E, 7E, 4H and 7I in the figure 2 may be the containers comprising the one or more reagents targeting the donor-informative polymorphisms.

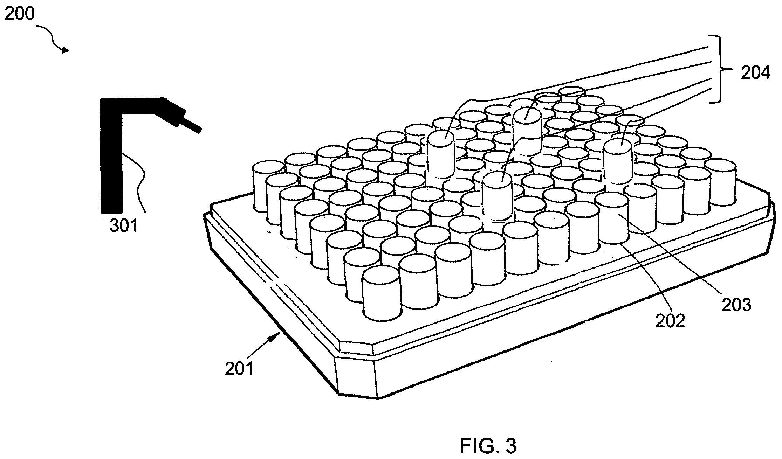

In examples, and with reference to figure 3, the system 200 may comprise an automation system, e.g., robotic pipette or other components such as a needle 301. The automation system can be configured to aspirate a volume of reagent from each container of the selection 204 of containers to perform the assay using the one or more reagents targeting the donor-informative polymorphisms. The automation system may receive the information relative to the selection of receptacles accommodating the selection 204 of containers.

In examples the system 200 further comprises an indicator system. In examples the indicator system is configured to relatively move the containers accommodated in the selection of receptacles with respect to the rest of containers; wherein the rest of containers comprise the containers accommodated in receptacles different from the selection of receptacles. The relative movement may comprise moving e.g., upwards or downwards, the selection 204 of containers or moving the rest of the containers. The relative movement advantageously indicates which containers are to be used for the assay so that errors in picking a wrong container or test tube is minimized. The indicator system may comprise an optical or visual indicator system, such as a laser, pointing at or illuminating each container of the selection 204 of containers, or mechanical actuators for gripping or picking each container of the selection 204 of containers or actuator for pushing each container of the selection 204 of containers.

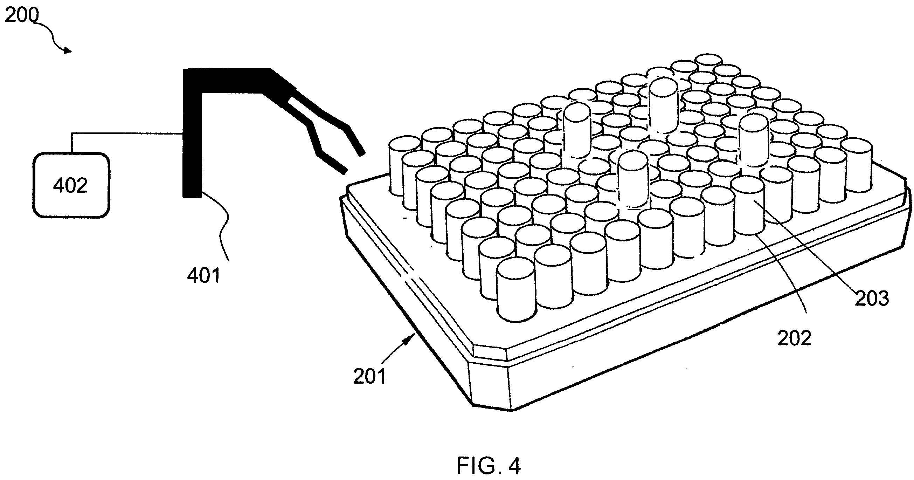

In examples, and with reference to figure 4, the system 200 may comprise an indicator system represented as a pickup device 401. The pickup device is configured for picking each container of the selection 204 of containers. The pickup device 401 may comprise one or more of a lamping or gripping or magnetic mechanism, a container handler, container picker, container gripper, container actuator, automated container manipulator, robotic container picker, or container extraction mechanism. Each of the containers in the selection 204 of containers may be partially or totally picked meaning that the selection 204 of containers may partially be moved with respect to the rest of containers or may be totally extracted from the test plate 201.

In examples the indicator system comprises a graphical interface configured to display the positions of the receptables so that a laboratory technician may, based on the display, manually pick or aspirate from the receptacle(s) of interest.

Figure 4 shows a non-limitative example in which the system comprising an interface 402, e.g., a USB interface or a communications module or a keyboard, configured to receive the information relative to the selection 204 of receptacles. The interface is communicatively coupled to the pickup device 401. The system of figure 3 may also comprise the interface 402 coupled to the robotic pipette 301. The interface 402 is configured to transmit the information relative to the received selection of receptacles accommodating the selection 204 of containers to the pickup device 401 or to the robotic pipette 301.

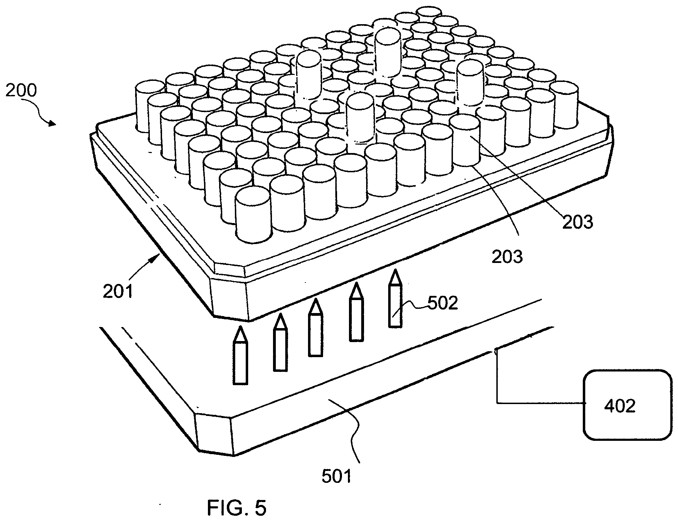

In examples, the indicator system comprises a pushing system 501, as shown in figure 5, for pushing the selection 204 of containers accommodated in the selection of receptacles. In examples, the pushing system 501 is configured to independently push each pushing element. In examples, at least a part of the pushing system 501 is integrated in the rack 201 and the pushing system comprises a plurality of pushing elements 502, wherein each of the pushing elements are in or directed to a receptacle. In examples, each pushing element 502 is configured to shift between a relaxed position and a pushing position for pushing the container. In the pushing position an upper portion of the pushed container(s) protrudes relative to the upper portion of the rest of plurality of containers. In figure 5, the relaxed position may be the position in which all the containers rest, and the pushing position may be the position in which the pushing elements 502 push the selection 204 of containers.

Figure 5 further shows the interface 402 communicatively coupled to the pushing system 501. The pushing system 501 is configured to receive the information relative to the selection of receptacles from the interface 402.

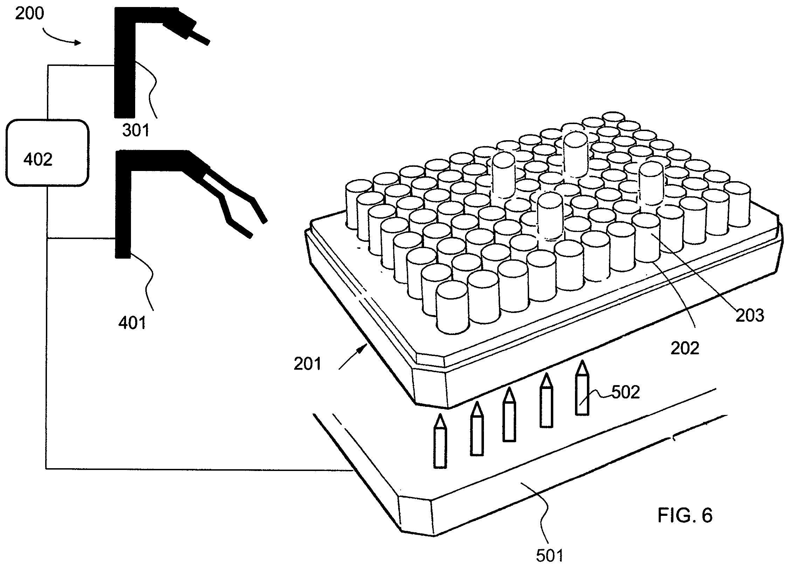

Figure 6 shows an example system comprising the test plate or rack 201 comprising the receptacles 202 and the containers 203, a robotic pipette 301, a pickup device 401, the pushing system and the interface 402. The interface 402 is communicatively coupled to the robotic pipette 301, to the pickup device 401 and to the pushing system 501.

In some examples the system 200 of any one of the examples comprises a robot or a robotic arm wherein the robot or the robotic arm comprises at least the pickup device 401 and/or the pushing system 501.

In some examples, the system 200 may comprise at least in part the kit or the device of the third aspect. In examples, the system 200 comprises means for carrying out a method as defined in any one of the first or second aspects.

In examples the system 200 may be configured to receive information about the presence and/or quantity of one or more donor-informative polymorphisms from a processor or from a computer system configured to implement any of the method steps of the CI methods described above.

In any of the methods, kits, assays, components, configurations, systems the graft may refer to tissue or an organ. In embodiments, the graft is an allograft. In embodiments, the graft is a xenograft. In embodiments, the graft is an organ, in particular, an organ selected from kidney, heart, liver, lung, pancreas, or intestine. In particular embodiments, the graft is a kidney.

### Numbered embodiments

For completeness the present disclosure further provides the following numbered embodiments.
1. An *in vitro* method for detecting donor-derived cell-free nucleic acids in a transplant recipient, said method comprising assaying at least one biological sample derived from the recipient containing cell-free nucleic acids to determine the presence and/or quantity of one or more markers in the genome of the recipient, wherein the one or more markers consist of one or more donor-informative polymorphisms, and wherein the presence of at least one donor-informative polymorphism is indicative of donor-derived cell-free nucleic acids in the transplant recipient.
2. The *in vitro* method according to embodiment 1, wherein the quantity of donor-derived cell-free nucleic acids is determined.
3. An *in vitro* method for determining graft health status in a transplant recipient, comprising assaying at least one biological sample derived from the recipient containing cell-free nucleic acids to quantify one or more markers in the genome of the recipient, wherein the one or more markers consist of one or more donor-informative polymorphisms, and outputting information about graft health status based on the quantity of at least one donor-informative polymorphism.
4. The *in vitro* method according to any one of embodiments 1-3, further comprise a prior step of identifying the donor-informative polymorphisms or, alternatively, of receiving the information about the donor-informative polymorphisms.
5. The *in vitro* method according to any one of embodiments 1-4, wherein the one or more donor-informative polymorphism is a copy number deletion (CND) comprising from 60 to 100,000 consecutive nucleotides, in particular, from 60 to 500 consecutive nucleotides, more in particular, from 200 to 500 consecutive nucleotides, which are present in the donor but deleted in the recipient.
6. The *in vitro* method according to any one of embodiments 1-4, wherein the one or more donor-informative polymorphism comprises an allele present in the genome of the donor but not in the genome of the recipient, said allele comprising at least 5 SNPs which are different in the donor and the recipient within a DNA polymorphic locus.
7. The *in vitro* method according to embodiment 5, wherein the DNA polymorphic locus is no longer than 120 nucleotides.
8. The *in vitro* method according to embodiment 7, wherein the DNA polymorphic locus is one that has sufficient genomic diversity to distinguish individuals within an ethnic group or population.
9. The *in vitro* method according to embodiment 8, wherein the DNA polymorphic locus is in the leukocyte antigen (HLA) region of the genome.
10. The *in vitro* method according to embodiment 9, wherein the DNA polymorphic locus is in the major histocompatibility complex (MHC) region of the genome.
11. The *in vitro* method according to any one of embodiments 6-10, wherein the polymorphism comprises from 5 to 10 SNPs.
12. The *in vitro* method according to any one of embodiments 1-11, wherein the assay is performed by using a quantitative or semiquantitative technique.
13. The *in vitro* method according to the previous embodiment, wherein the assay is performed by dPCR.
14. The *in vitro* method according to any one of embodiments 1-13, wherein from 2 to 20 donor-informative polymorphisms are determined, in particular from 4 to 16.
15. The *in vitro* method according to the previous embodiment, wherein the donor-informative polymorphisms are determined cumulatively in a single reaction.
16. The *in vitro* method according to embodiment 14, wherein the donor-informative polymorphisms are determined in more than one reaction.
17. The *in vitro* method according to any one of embodiments 14-16, wherein the donor-informative polymorphisms are determined in the same detection channel.
18. The *in vitro* method according to any one of embodiments 14-16, wherein the donor-informative polymorphisms are determined in different detection channels.
19. The *in vitro* method according to any one of embodiments 1-18, wherein the assay is performed using reagents comprising primers and/or probes targeting the donor-informative polymorphisms.
20. The *in vitro* method according to the previous embodiment, wherein the primers are configured to amplify a region no longer than 200 nucleotides, in particular, no longer than120 nucleotides, more in particular, around 60 nucleotides.
21. The *in vitro* method according to the previous embodiment, wherein the probes are labelled and configured to bind the amplicons.
22. The *in vitro* method according to the any one of embodiments 19-21, wherein the primers are configured to amplify a DNA polymorphic locus no longer than 120 nucleotides comprising at least five SNPs.
23. The *in vitro* method according to the preceding embodiment, wherein the probes are labelled and are configured to bind only to amplicons containing the at least five SNPs.
24. The *in vitro* method according to any one of embodiments 19-23, wherein the assay is performed further using labelled reagents targeting a control sequence.
25. The *in vitro* method according to any one of embodiments 19-23, wherein the labels in the reagents are the same.
26. The *in vitro* method according to any one of embodiments 19-23, wherein the labels in the reagents are different.
27. The *in vitro* method according to any one of embodiments 19-26, wherein the labels are fluorescent molecules.
28. The *in vitro* method according to any one of embodiments 1-27, wherein the donor-informative polymorphisms are previously identified by comparing the genome of the donor with that of the recipient, for example from analysing existing genotyping data, wherein the donor-informative polymorphisms are identified as those which distinctive for the donor with respect to the recipient.
29. The *in vitro* method according to any one of embodiments 1-28, wherein the donor-informative polymorphisms are identified in a prior assay.
30. The *in vitro* method according to the preceding embodiment, wherein the donor-informative CNDs and are identified by (a) performing a prior assay on at least one biological sample derived from the recipient containing cell-free nucleic acids to determine a plurality of CNDs, and (b) identifying the donor-informative CNDs as a subset of the plurality of CNDs which are determined to be present in the donor but deleted in the recipient.
31. The *in vitro* method according to the preceding embodiment, wherein the plurality of CNDs comprises from 60 to 100,000 consecutive nucleotides, in particular, from 200 to 500 consecutive nucleotides.
32. The *in vitro* method according to any one of embodiments 30-31, wherein the CNDs are characterised by being found in a frequency ranging from 40 to 60% of a population.
33. The *in vitro* method according to any one of embodiments 30-32, wherein the prior assay comprises determining from 10 to 100 CNDs, in particular from 20 to 60 CNDs, more particularly from 30 to 60 CNDs.
34. The *in vitro* method according to any one of embodiments 30-33, wherein the prior assay is performed by using a quantitative or semiquantitative analytical technique.
35. The *in vitro* method according to the previous embodiment, wherein the analytical technique is dPCR.
36. The *in vitro* method according to any one of embodiments 30-35, wherein the assay is performed using reagents comprising primers and/or probes targeting the plurality of CNDs and, optionally, control sequences.
37. The *in vitro* method according to the preceding embodiment, wherein the primers targeting the CNDs are configured to amplify a region no longer than 200 nucleotides, in particular, no longer than 120 nucleotides.
38. The *in vitro* method according to the preceding embodiment, wherein the primers targeting the CNDs are configured to amplify a region from 60 to 90 nucleotides, in particular around 60 nucleotides.
39. The *in vitro* method according to any one of embodiments 36-38, wherein the probes are labelled and configured to bind the amplicons.
40. The *in vitro* method according to the preceding embodiment, wherein the labels are fluorescent molecules.
41. The *in vitro* method according to the preceding embodiment, wherein two or more, for example 4, different CNDs and a control sequence are determined in a single reaction using differently-labelled reagents for each different CND and the control sequence.
42. The *in vitro* method according to the preceding embodiment, wherein the biological sample derived from a recipient is aliquoted into 12 sample aliquots, each for determining two or more, for example 4, different CNDs and a control sequence in a single reaction using differently-labelled reagents for each different CND and the control sequence.
43. The *in vitro* method according to any one of embodiments 33-42, wherein identifying the donor-informative CNDs comprises identifying the CNDs present at a level below a certain reference value or within a range of reference values that indicates that it is present in the donor and deleted in the recipient.
44. The *in vitro* method according to the preceding embodiment, wherein when the concentration of a CND is determined to be below 100 copies per ml sample derived from the recipient, this is indicative of the CNDs being present in the donor and deleted in the recipient for kidney, heart, or lung recipients, or alternatively, when a concentration of a CND is determined to be below 400 copies per ml sample derived from the recipient, this is indicative of the CNDs being present in the donor and deleted in the recipient for in liver recipients.
45. The *in vitro* method according to any one of embodiments 33-44, wherein one of the following situations are found for each CND of the plurality when performing the prior assay with dPCR:
   (i) no target CND , meaning that said CND is deleted in the donor and in the recipient; or
   (ii) a first quantity of the target DNA, said first quantity being indicative of the CND being present (not deleted) in the recipient; or
   (iii) a second quantity of the target DNA, said second quantity being below a reference value or within a range of reference values which is indicative of the CND being deleted in the recipient but present in the donor, for example the second quantity being below 0.2%, 1%, 2%, 5%, 10%, or 20% or more of the most abundant CNDs detected in the assay, and
   the target CND is identified as donor-informative when the number of copies determined for said CND is the second number.
46. The *in vitro* method according to any one of embodiments 1-45, wherein the biological sample derived from the recipient is selected from the group consisting of serum, pus, respiratory fluid, lymph fluid, faeces, bile, saliva, sputum, semen, vaginal flow, cerebrospinal fluid, brain fluid, ascites, milk, secretions from the genitourinary tract, and combinations thereof, in particular selected from blood, plasma, urine, or bronchoalveolar lavage.
47. The *in vitro* method according to the preceding embodiment, wherein the biological sample is plasma.
48. The *in vitro* method according to the any one of embodiments 46-47, wherein more than one biological sample is used for the assays.
49. The *in vitro* method according to the any one of embodiments 46-48, wherein cell-free nucleic acids are extracted from the sample(s) and used for performing the assay(s).
50. The in vitro method according to any one of embodiments 1-49, wherein the quantity of donor-derived cell-free nucleic acids in the recipient is positively correlated to an extent of graft injury.
51. The in vitro method according to any one of embodiments 1-50, wherein a quantity of donor-derived cell-free nucleic acids above a reference is indicative of graft rejection.
52. The in vitro method according to any one of embodiments 3-50, wherein the outputted information provided comprises screening for graft rejection, risk of suffering from graft rejection, monitoring of graft rejection, determining the efficacy of a therapy for graft rejection, diagnosis of graft rejection, prognosis of graft rejection, diagnosis of viral infections, prognosis of viral infections, monitoring of viral infections, determining the efficacy of a therapy for viral infections, suggestions on additional testing such as biopsy, or a combination thereof.
53. A method for administering an appropriate medical regime (therapy) for a transplant recipient, comprising determining graft health status as defined in any one of embodiments 3-52, and recommending or administering an appropriate medical regime when determining that the graft is damaged.
54. A kit or device comprising means for carrying out a method as defined in any one of embodiments 1-53, said means comprising:
   (i) a first plurality of containers containing labelled reagents for determining a plurality of polymorphisms, each container of the first plurality containing labelled reagents directed to more than one polymorphism and wherein each container of the first plurality contains differently labelled reagents directed to each different polymorphism.
55. A kit or device comprising means for carrying out a method as defined in any one of embodiments 1-53, said means comprising:
   (ii) a second plurality of containers containing labelled reagents for determining the plurality of polymorphisms as defined in (i), each container of the second plurality containing labelled reagents directed to a different polymorphism, wherein the labels are the same or different.
56. The kit or device according to the preceding embodiment, wherein each container of the first plurality contains labelled reagents targeting 4-6 polymorphisms, wherein each recipient of the first plurality contains differently labelled reagents targeting each different polymorphism.
57. The kit or device according to the preceding embodiment, further comprising reagents targeting one or more control sequences.
58. The kit or device according to any one of embodiments 54-57, wherein the reagents are a plurality of oligonucleotide sequences or complements thereof directed at polymorphisms, or to a control sequence (such as a housekeeping gene).
59. The kit or device according to any one of embodiments 54-58, wherein the polymorphisms are CNDs.
60. The kit or device according to any one of embodiments 54-58, wherein the polymorphisms are alleles present in the genome of the donor but not in the genome of the recipient, said alleles comprising at least 5 SNPs which are different in the donor and the recipient within a DNA polymorphic locus no longer than 120 nucleotides, in particular, wherein said DNA polymorphic locus is within an HLA region, for example, within the leukocyte antigen (HLA) region of the genome.
61. The kit or device according to any one of embodiments 54-60, wherein the reagents comprise primers and labelled probes.
62. The kit or device according to the preceding embodiment, wherein the primers are configured to amplify a region no longer than 200 nucleotides, in particular no longer than 120 nucleotides, more in particular around 60 nucleotides, wherein the probes are labelled and configured to bind the amplicons.
63. The kit or device according to the any one of embodiments 61-62, wherein the primers are configured to amplify a DNA polymorphic locus comprising at least five SNPs.
64. The kit or device according to the any one of embodiments 61-62, wherein the probes are configured to bind only to amplicons containing the at least five single nucleotide substitutions and/or deletions.
65. The kit or device according to any one of embodiments 54-64, which is compatible with digital PCR.
66. A computer-implemented method, 100, for detecting donor-derived cell-free nucleic acids in a transplant recipient, the CI method comprising
   - instructing 101 assay performance, the assay comprising assaying at least one biological sample derived from the recipient containing cell-free nucleic acids to determine a presence and/or a quantity of one or more markers, wherein the one or more markers consist of one or more donor-informative polymorphisms;
   - receiving 102 the presence and/or quantity of one or more donor-informative polymorphisms in the biological sample; and
   - providing 103 result information about donor-derived cell-free nucleic acids in the transplant recipient.
67. The CI method according to the preceding embodiment, wherein the one or more donor-informative polymorphisms are selected from:
   (i) a copy number deletion (CND) comprising from 60 to 100,000 consecutive nucleotides which is present in the donor but deleted in the recipient, or
   (ii) an allele present in the genome of the donor but not in the genome of the recipient, said allele comprising at least 5 SNPs which are different in the donor and the recipient within a DNA polymorphic locus no longer than 120 nucleotides, in particular wherein said DNA polymorphic locus is within a HLA region.
68. The CI method according to any one of embodiments 66-67, wherein instructing assay performance is implemented by instructing assay performance to a system performing the assay, for example, to a thermocycler or to a digital PCR system.
69. The CI method according to any one of embodiment 66-68, wherein instructing the assay performance comprises instructing a thermocycler or to a digital PCR system to perform PCR on the biological sample using reagents targeting the one or more donor-informative polymorphisms.
70. The CI method according to any one of embodiments 66-69, further comprising receiving the presence and/or quantity of one or more donor-informative polymorphisms from a system performing the assay, for example, from a thermocycler or from a digital PCR system.
71. The CI method according to any one of embodiments 66-70, further comprising providing result information about the donor-derived cell-free nucleic acids in the transplant recipient to a user interface, e.g., a graphical interface.
72. The CI method according to any one of embodiments 66-71, wherein the donor-informative polymorphisms (e.g. CNDs or alleles comprising SNPs as defined above) are received from a prior assay, from a database, from analysing existing genotyping data of the donor and the recipient, or from genotyping.
73. The CI method according to any one of embodiments 66-72, further comprising instructing a prior assay performance on a biological sample containing cell-free nucleic acids obtained from the recipient to determine a first plurality of CNDs.
74. The CI method according to embodiment 73, further comprising selecting one or more donor-informative CNDs as a subset of the plurality of CNDs which are present in the genome of the donor but deleted from the genome of the recipient to generate one or more reagents targeting the one or more donor-informative CNDs.
75. The CI method according to embodiment 73 or 74 further comprising obtaining one or more reagents targeting the one or more donor-informative CNDs, e.g., primers and labelled probes targeting the one or more donor-informative CNDs.
76. The CI method according to any one of embodiments 73-75, wherein instructing the prior assay performance comprises instructing a digital PCR system or a thermocycler to perform PCR on a biological sample.
77. The CI method according to any one of embodiments 74-76, wherein selecting the one or more donor-informative CNDs comprises selecting a subset of the plurality of CNDs, wherein each CND of the subset is selected based on a detectable signal resulting from the prior assay which indicates that the CND is present in the donor and deleted in the recipient.
78. The CI method according to the preceding embodiment, wherein the detectable signal resulting from the prior assay is correlated to an amount of target CND, which, in turn indicates that it is donor-derived, for example, the amount of target CND is below a certain reference value or within a range of reference values that indicates that the target CND is donor-derived.
79. The CI method according to the preceding embodiment, further comprising comparing the amount of the target CND with a reference value or range of reference values to determine whether the target CND belongs to the donor.
80. The CI method according to any one of embodiments 66-79, further comprising sending information relative to a selection of receptacles of a plurality of receptacles to a system as defined in the disclosure, the selection of receptables containing reagents targeting the one or more donor-informative polymorphisms (e.g. CNDs or alleles comprising SNPs as defined in embodiment 67 (ii)).
81. The CI method according to the preceding embodiment, wherein sending information comprises sending information relative to the selection of receptacles which are correlated with the plurality of polymorphisms, e.g., CNDs or alleles comprising SNPs as defined in embodiment 67 (ii), of the reagents targeting the one or more donor-informative polymorphisms (e.g. CNDs or alleles comprising SNPs as defined in embodiment 67 (ii)).
82. The CI method according to any one of embodiments 80-81, wherein the information relative to the selection of receptacles is based on a table identifying the positions of a test plate or rack 201, as defined in the disclosure, occupied by containers including the reagents targeting the one or more donor-informative polymorphisms (e.g. CNDs or alleles comprising SNPs as defined in embodiment 67 (ii)).
83. The CI method according to any one of embodiments 80-82, further comprising outputting information about the selection of receptacles via a user interface, e.g., a graphical interface.
84. The CI method according to any one of embodiments 80-83, further comprising detecting the presence and/or quantity of donor-derived cell-free nucleic acids in the recipient.
85. The CI method according to any one of embodiments 66-84, further comprising determining and/or providing or outputting result information about a graft health status based on the detection of the presence and/or quantity of donor-derived cell-free nucleic acids in the recipient, e.g., on a user interface, or on a graphical interface.
86. The CI method according to the preceding embodiment, wherein the outputted information comprises screening for graft rejection, risk of suffering from graft rejection, monitoring of graft rejection, determining the efficacy of a therapy for graft rejection, diagnosis of graft rejection, prognosis of graft rejection, diagnosis of viral infections, prognosis of viral infections, monitoring of viral infections, determining the efficacy of a therapy for viral infections, suggestions on additional testing such as biopsy, or a combination thereof.
87. The CI method according to any one of embodiments 85-86, further comprising determining and/or providing or outputting information about an appropriate medical regime for the recipient based on the detection of the presence and/or quantity of donor-derived cell-free nucleic acids in the recipient.
88. A system 200 for preparation of one or more reagents targeting at least one donor-informative polymorphism,
   - the system comprising a test plate or rack 201 comprising a plurality of receptacles 202, each receptacle of the plurality of receptacles configured to accommodate a container 203; and
   - wherein the system 200 is configured to receive information relative to a selection of receptacles of the plurality of receptacles wherein the selection of receptacles are configured to accommodate at least a selection 204 of containers, the selection of containers to comprise one or more reagents targeting one or more donor-informative polymorphisms.
89. The system 200 according to embodiment 88, wherein the one or more donor-informative polymorphisms are identified as described in any one of the embodiments defined above.
90. The system 200 according to embodiment 88 or 89, wherein the reagents comprise primers and labelled probes targeting the one or more donor-informative polymorphisms.
91. The system 200 according to any one of embodiments 88-90, further configured to:
   - obtain or receive at least the one or more reagents targeting the one or more donor-informative polymorphisms, for example the system comprising and using a reagents input, feeder, loading station, loader, cartridge feeder, cassette, supply module, input tray, or input module, and
   - to prepare the one or more reagents into one or more containers, for example the system comprising and using one or more of: robotic arms, pipettes or a combination thereof to, e.g., insert or add one or more reagents into one or more containers.
92. The system 200 according to any one of embodiments 88-91, wherein each receptacle of the plurality of receptacles 202 accommodates a container 203, particularly wherein each container 203 contains reagents directed to a single polymorphism of a plurality of polymorphisms as defined in any one of embodiments 29-33.
93. The system 200 according to any one of embodiments 88-92, further comprising at least the selection 204 of containers.
94. The system 200 according to any one of embodiments 88-93, wherein the test plate or rack 201 comprises a matrix of receptables organized by:
   - rows, e.g, labelled as 1...8, of receptacles and
   - columns, e.g, labelled as A...L, of receptacles;
   - where each position of the rack 201 is labeled by its position in the respective row and columns, for example, receptacle 5B.
95. The system 200 according to any one of embodiments 88-94, further comprising an automation system, e.g., robotic pipette or other components such as a needle 301, wherein the automation system is configured to aspirate a volume of probe from each container of the selection 204 of containers for assay performance using one or more reagents targeting the one or more donor-informative polymorphisms.
96. The system 200 according to the preceding embodiment, wherein the automation system is configured to receive information relative to a selection of receptacles accommodating the selection 204 of containers.
97. The system 200 according to any one of embodiments 88-96, further comprising an indicator system.
98. The system 200 according to the preceding embodiment, wherein the indicator system is configured to relatively move the containers accommodated in the selection 204 of receptacles with respect to the rest of containers; wherein the rest of containers comprise the containers accommodated in receptacles different from the selection of receptacles, wherein the relative movement may comprise moving e.g., upwards or downwards, the selection 204 of containers or moving the rest of the containers.
99. The system 200 according to embodiment 97 or 98, wherein the indicator system comprises one or more of:
   - an optical or visual indicator system, such as a laser, configured to pointing at or to illuminate each container of the selection 204 of containers,
   - mechanical actuators for gripping or picking each container of the selection 204 of containers or actuator for pushing each container of the selection 204 of containers.
100. The system 200 according to any one of embodiments 97-99, wherein the indicator system is a pickup device 401, wherein the pickup device is configured to pick each container of the selection 204 of containers.
101. The system 200 according to the preceding embodiment, wherein the pickup device 401 comprises one or more of a lamping or gripping or magnetic mechanism, a container handler, container picker, container gripper, container actuator, automated container manipulator, robotic container picker, container extraction mechanism.
102. The system 200 according to any one of embodiments 97-101, wherein the indicator system comprises a graphical interface configured to display the positions of the receptables.
103. The system 200 of any one of embodiments 88-102, comprising an interface 402, or input interface, e.g., a USB interface or a communications module or a keyboard, configured to receive the information relative to the selection 204 of receptacles.
104. The system 200 of the preceding embodiment, wherein the interface is communicatively coupled to the pickup device 401.
105. The system 200 of any one of embodiments 103-104, wherein the interface 402 is coupled to the robotic pipette 301.
106. The system 200 of any one of embodiments 103-105, wherein the interface 402 is configured to transmit information relative to the received selection of receptacles accommodating the selection 204 of containers to the pickup device 401 or to the robotic pipette 301.
107. The system 200 of any one of embodiments 97-106, wherein the indicator system comprises a pushing system 501 configured to push the selection 204 of containers accommodated in the selection of receptacles.
108. The system 200 of embodiment 107, wherein the pushing system 501 is configured to independently push each pushing element.
109. The system 200 of embodiment 107 or 108, wherein at least a part of the pushing system 501 is integrated in the rack 201 and the pushing system comprises at least one of pushing element 502, wherein each pushing element is in or directed to a receptacle.
110. The system 200 of embodiment 109, wherein each pushing element 502 is configured to shift between a relaxed position and a pushing position for pushing a container, wherein
   - in the pushing position an upper portion of the pushed container(s) protrudes relative to the upper portion of the rest of plurality of containers and
   - wherein, in the relaxed position, all the containers rest.
111. The system 200 of any one of embodiments 103-110 wherein the interface 402 is communicatively coupled to the pushing system 501 and wherein the pushing system 501 is configured to receive information relative to the selection of receptacles from the interface 402.
112. The system 200 of any one of embodiments 88-111, comprising:
   - the test plate or rack 201 comprising the receptacles 202 and the containers 203,
   - a robotic pipette 301,
   - a pickup device 401,
   - a pushing system and
   - the interface 402 communicatively coupled to the robotic pipette 301, to the pickup device 401 and to the pushing system 501.
113. The system 200 of any one of embodiments 88-112, comprising a robot or a robotic arm wherein the robot or the robotic arm comprises at least the pickup device 401 and/or a pushing system 501.
114. The system 200 of any one of embodiments 88-113, comprising at least in part the kit of any one of embodiment 54- 65.
115. The system 200 of any one of embodiments 88-114, comprising means for carrying out a CI method of any one of embodiments 66-87.
116. The system 200 of any one of embodiments 88-115, further configured to receive information about the presence and/or quantity of one or more donor-informative polymorphisms from a processor or from a computer system configured to implement any of the method steps of the CI method of any one of embodiments 66-87.
117. An in vitro method for detecting donor-derived cell-free nucleic acids in a transplant recipient, said method comprising assaying at least one biological sample derived from the recipient containing cell-free nucleic acids to detect the presence and/or quantity of two or more donor-informative polymorphisms in a single reaction using labelled reagents directed at said donor-informative polymorphisms, wherein all labelled reagents contain the same detectable label, wherein detecting at least one donor-informative polymorphism is indicative of donor-derived cell-free nucleic acids in the transplant recipient.
118. The in vitro method according to embodiment 117, wherein the donor-informative polymorphisms are selected from the group consisting of:
   (i) a copy number deletion (CND) comprising from 60 to 100,000 consecutive nucleotides which is present in the donor but deleted in the recipient, or
   (ii) an allele present in the genome of the donor but not in the genome of the recipient, said allele comprising at least 5 SNPs which are different in the donor and the recipient within a DNA polymorphic locus no longer than 120 nucleotides, in particular wherein said DNA polymorphic locus is within a HLA region.

Throughout the description, example, embodiments and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

Example 1. The following is an example of detecting donor derived cell-free DNA in a transplant recipient with the method of the disclosure.
1. Prior assay to identify donor-informative CNDs.
1.1. Cell-free DNA is extracted from plasma obtained from a recipient. The plasma is obtained from a standard 10 ml whole blood extraction (4 ml plasma is obtained).
1.2. The cell-free DNA is aliquoted in 12 test tubes, each test tube containing primers and fluorescent probes for targeting **4** different CNDs and a housekeeping gene (control). In total, a panel of 48 CNDs is screened. In each tube, each probe targeting a different CND is labelled with a different fluorophore. The probe targeting the housekeeping gene is also labelled with a different fluorophore.
1.3. dPCR is performed on each of the 12 test to quantify the 48 CNDs and the housekeeping genes.
1.4. From the results of the dPCR, a number « n » of donor informative CNDs were selected. The donor informative CNDs are those present in the donor and deleted in the recipient. The following criteria is applied:
   - When both the recipient and the donor are nullisomic for a CND: no amplification of target sequence takes place; no signal is retrieved; the dPCR results in negligible concentration for this sequence. This CDN is not informative and is thus not selected.
   - When none of the recipient and the donor are nullisomic for a CND: amplification of target sequence takes place from both donor and recipient cell-free DNA; a very high signal is retrieved; the dPCR results in a high concentration for this sequence. This CDN is not informative and is thus not selected.
   - When the donor is nullisomic for a CND and the recipient is not: amplification of target sequence takes place from recipient cell-free DNA; a very high signal is retrieved; the dPCR results in a high concentration for this sequence. This CDN is not informative and is thus not selected.
   - When the recipient is nullisomic for a CND and the donor is not: amplification of target sequence takes place only from donor cell-free DNA; since the amount of donor-derived cell-free DNA is very low compared to that coming from the recipient, a signal is retrieved, but this is a weak signal; the dPCR results in a concentration for this sequence that is below a reference value or within a range of reference values that indicates that it comes from the donor. This CDN is informative and is thus selected.
2. Assay to quantify donor-derived cell-free nucleic acids.
2.1. Reagents (primers and fluorescent probes) targeting each of the « n » donor-informative CNDs found in the prior assay are placed in a single test tube. Reagents (primers and fluorescent probes) targeting a housekeeping gene are added to the single test tube. Cell-free DNA is extracted from a second plasma sample obtained from the same or a second standard blood extraction from the patient. The cell-free DNA extracted from the second sample is placed in one or more test tubes containing the reagents targeting the donor-informative CNDs and reagents targeting the housekeeping gene. In a particularly advantageous configuration, the whole volume of cell-free DNA extracted from the second sample is placed in a single test tube containing the reagents targeting the donor-informative CNDs and reagents targeting the housekeeping gene. The fluorescence of each probe targeting each donor-informative CNDs may be the same or different from the rest. However, the fluorescence of the probe targeting the housekeeping gene is different from the others.
2.2. dPCR is performed to cumulatively quantify the « n » donor-informative CNDs and the housekeeping gene. In a particular configuration, all the « n » donor-informative CNDs and the housekeeping gene are quantified in a single reaction in the same test tube.
2.3. The dPCR provides quantitative information for the « n » donor-informative CNDs. Since all these markers are informative, i.e. they all come from the donor, the cumulative concentration determined corresponds to donor derived cell-free nucleic acids present in the sample derived from the recipient.

### Example 2

Donor-derived cell-free nucleic acids were determined with dPCR by following the assay described in point 2 of example 1. The donor-informative CNDs had been previously identified. The donor-informative CNDs are coded M004, M032, M016, M043, M003 and M006 (each code corresponding to a different donor-informative CDN). The assay also determined a housekeeping sequence, which is coded ACE. M003 and M006 were determined individually into independent reactions (separate reaction vessels). M004 and M032 were determined together in a single reaction (same reaction vessel). M016 and M043 were determined together in another single reaction.

Results show higher copies/µL are determined when two donor-informative markers are combined into one reaction (table 3).

**Table 3. Results obtained by dPCR for the assayed targets**

| **RUN1** | **RUN2** | **RUN3** | **RUN4** | | | | |
|---|---|---|---|---|---|---|---|
| Target | Copies/µL | Copies/µL | Copies/µL | Copies/µL | Average | std | cv |
| M004+M032 | 8 | 7 | 9 | 13 | 9,25 | 2,28 | 24,62% |
| M016+M043 | 6 | 4 | 8 | 10 | 7,00 | 2,24 | 31,94% |
| ACE | 3057 | 3140 | 3045 | 3158 | | | |
| M003 | 4 | 5 | 4 | 8 | 5,25 | 1,64 | 31,23% |
| M006 | 5 | 3 | 2 | 4 | 3,50 | 1,12 | 31,94% |
| M004+M032 | 4 | 7 | 15 | 15 | 10,25 | 4,87 | 47,48% |
| M016+M043 | 6 | 5 | 3 | 6 | 5,00 | 1,22 | 24,49% |
| ACE | 3026 | 3201 | 3098 | 3038 | | | |
| M003 | 2 | 5 | 6 | 1 | 3,50 | 2,06 | 58,90% |
| M006 | 2 | 4 | 5 | 4 | 3,75 | 1,09 | 29,06% |
| M004+M032 | 16 | 11 | 4 | 6 | 9,25 | 4,66 | 50,35% |
| M016+M043 | 3 | 4 | 15 | 9 | 7,75 | 4,76 | 61,46% |
| ACE | 3010 | 3130 | 3178 | 3112 | | | |
| M003 | 2 | 4 | 7 | 6 | 4,75 | 1,92 | 40,43% |
| M006 | 2 | 6 | 6 | 4 | 4,50 | 1,66 | 36,85% |
| M004+M032 | 13 | 15 | 12 | 8 | 12,00 | 2,55 | 21,25% |
| M016+M043 | 5 | 7 | 9 | 6 | 6,75 | 1,48 | 21,91% |
| ACE | 3231 | 3219 | 3188 | 3280 | | | |
| M003 | 1 | 3 | 6 | 6 | 4,00 | 2,12 | 53,03% |
| M006 | 2 | 10 | 4 | 4 | 5,00 | 3,00 | 60,00% |

### Example 3.

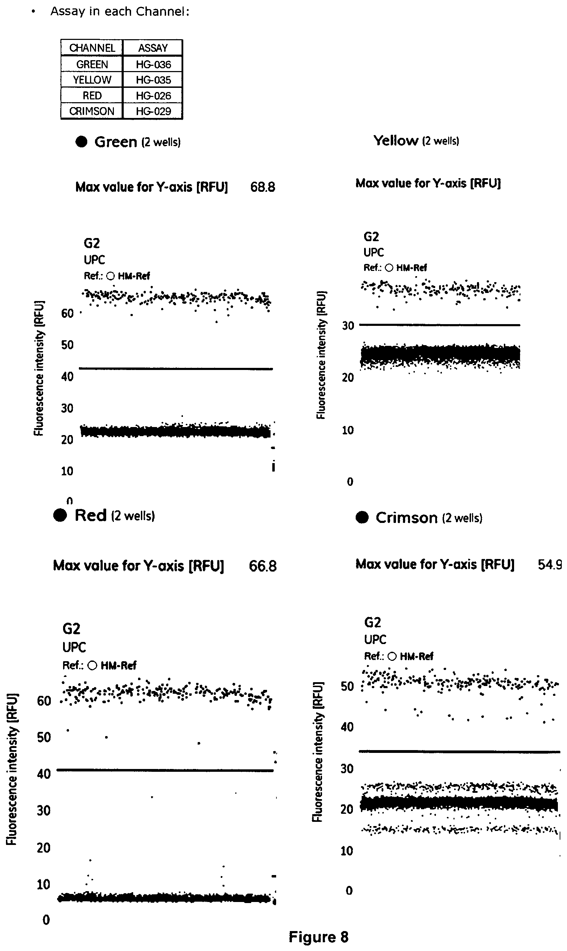

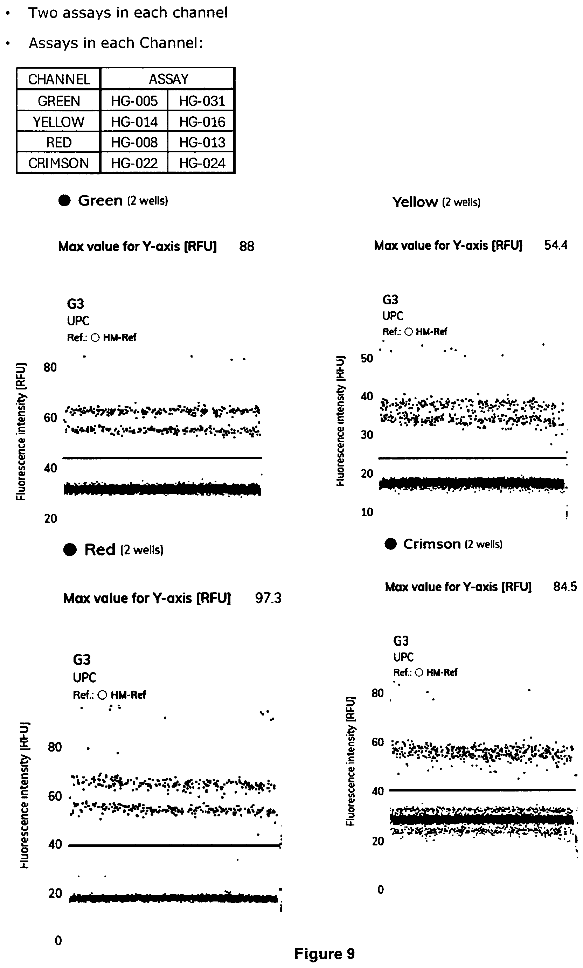

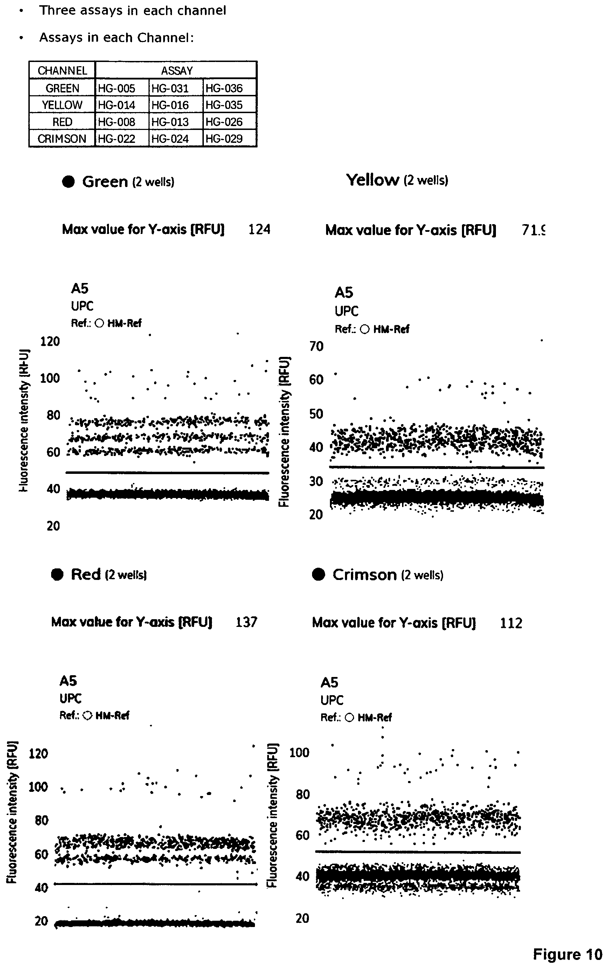

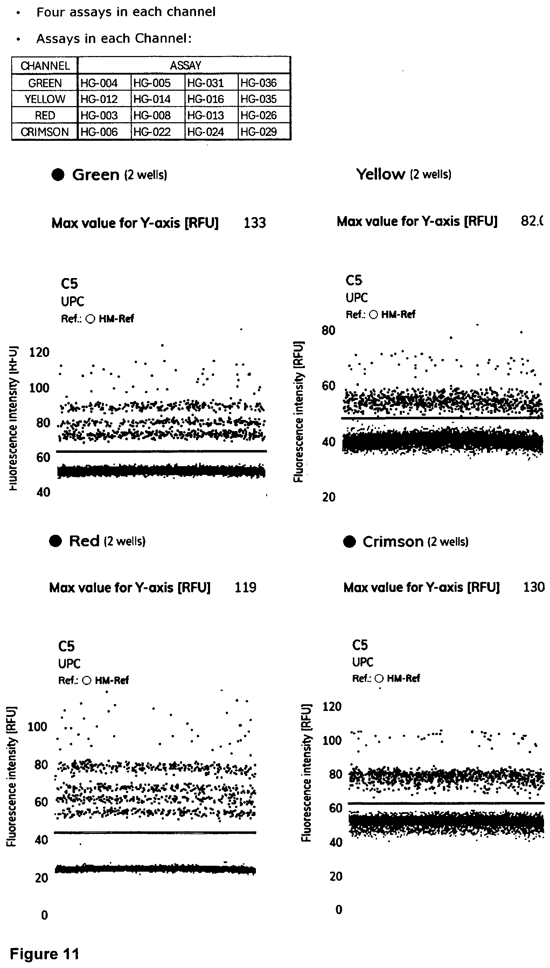

Several assays were carried out with dPCR to determine donor-derived cell-free nucleic acids by following the assay described in point 2 of example 1. The donor-informative CNDs had been previously identified. The donor-informative CNDs are coded HG-XXX (for example, HG-004, HG-005, HG-036, etc., wherein each code corresponds to a different donor-informative CDN). Each experiment was configured to detect the different CNDs in the same or in different detection channels. Results are shown in figures 8-11. It may be observed that donor-informative CNDs are correctly quantified by dPCR. Additionally, it has been shown that in some cases it was possible to differentially quantify donor-informative CNDs even when they are detected in the same channel.

### Citation List

WO2013049892
Conrad et al. (2010) Nature 464: 704-712
McCarroll et al. (2008) Nat Genet 40(10): 1 166-1 174
Burtis et al., 2008, Chapter 14, section "Statistical Treatment of Reference Values"
Whitlam et al. American Journal of Transplantation, Volume 19, Issue 4, 1037 - 1049
Aubert et al. (2024) Nat Med 30:2320-2327

## Claims

1. An *in vitro* method for detecting donor-derived cell-free nucleic acids in a transplant recipient, said method comprising assaying at least one biological sample derived from the recipient containing cell-free nucleic acids to detect a presence and/or a quantity of one or more markers, wherein the one or more markers consist of one or more donor-informative polymorphisms selected from:
(i) a copy number deletion (CND) comprising from 60 to 100,000 consecutive nucleotides which are present in the genome of the donor but deleted in the genome of the recipient, or
(ii) an allele present in the genome of the donor but not in the genome of the recipient, said allele comprising at least 5 SNPs which are different in the donor and the recipient within a DNA polymorphic locus no longer than 120 nucleotides, in particular wherein said DNA polymorphic locus is within a HLA region, and
wherein the presence and/or quantity of at least one donor-informative polymorphism is indicative of donor-derived cell-free nucleic acids in the transplant recipient.

2. An *in vitro* method for determining graft health status in a transplant recipient, said method comprising assaying at least one biological sample derived from the recipient containing cell-free nucleic acids to quantify one or more markers, wherein the one or more markers consist of one or more donor-informative polymorphisms selected from:
(i) a copy number deletion (CND) comprising from 60 to 100,000 consecutive nucleotides which are present in the genome of the donor but deleted in the genome of the recipient , or
(ii) an allele present in the genome of the donor but not in the genome of the recipient, said allele comprising at least 5 SNPs which are different in the donor and the recipient within a DNA polymorphic locus no longer than 120 nucleotides, in particular wherein said DNA polymorphic locus is within a HLA region,
and outputting information about graft health status based on the quantity of at least one donor-informative polymorphism.

3. The *in vitro* method according to any one of claims 1-2, wherein the quantity of donor-derived cell-free nucleic acids is detected by performing the assay using digital PCR (dPCR).

4. The *in vitro* method according to any one of claims 1-3, further comprising a prior step of identifying the donor-informative polymorphisms or, alternatively, receiving the information about the donor-informative polymorphisms.

5. The *in vitro* method according to any one of claims 1-4, wherein the assaying is performed by dPCR using primers and labelled probes targeting the donor-informative polymorphisms, in particular, wherein the primers are configured to amplify a region no longer than 120 nucleotides.

6. The *in vitro* method according to any one of claims 1-5, wherein the assaying comprises detecting the presence and/or quantity from 4 to 20 donor-informative polymorphisms.

7. The *in vitro* method according to any one of claims 1-6, wherein the assaying comprises detecting the presence and/or quantity of at least 4 donor-informative polymorphisms in a single reaction using labelled reagents directed at said donor-informative polymorphisms, wherein the labels are the same or different, and wherein the donor-informative polymorphisms are not individually detected.

8. The in vitro method according to any one of claims 4-7, wherein the donor-informative CNDs are identified by:
(a) performing a prior assay on at least one biological sample derived from the recipient containing cell-free nucleic acids to detect a plurality of CNDs, wherein the CNDs are **characterised by** each comprising from 60 to 100,000 consecutive nucleotides and by each being found in a frequency ranging from 40 to 60% of a population; and
(b) identifying the donor-informative CNDs as a subset of the plurality of CNDs which are detected to be present below a reference value or within a range of reference values which is indicative of the CNDs being present in the donor and deleted in the recipient.

9. The in vitro method according to claim 8, wherein:
a) the prior assay comprises performing dPCR, thereby detecting for each target CND:
(i) no target CND , meaning that said CND is deleted in the donor and in the recipient; or
(ii) a first quantity of the target DNA, said first quantity being indicative of the CND being present in the recipient; or
(iii) a second quantity of the target DNA, said second quantity being below a reference value or within a range of reference values which is indicative of the CND being deleted in the recipient but present in the donor, for example the second quantity being below 20% of the most abundant CNDs detected in the assay, and
b) identifying the target CND as donor-informative when the quantity detected for said CND is the second quantity.

10. The *in vitro* method according to any one of claims 8-9, wherein the prior assay comprises detecting more than one different CND in the same reaction using differently-labelled reagents for each different CND.

11. The *in vitro* method according to any one of claims 3-7, wherein the donor-informative polymorphisms are identified by genotyping of the donor and the recipient, or alternatively by receiving and analysing existing genotyping data of the donor and recipient.

12. The *in vitro* method according to any one of claims 1-11, wherein the biological sample(s) is(are) selected from the group consisting of blood, plasma, urine, or bronchoalveolar lavage.

13. A kit or device comprising means for carrying out a method as defined in any one of claims 1-12, said means comprising:
(i) a first plurality of containers containing labelled reagents for detecting a plurality of polymorphisms, each container of the first plurality containing labelled reagents directed to more than one polymorphism, for example, at least 4 polymorphisms, and wherein each recipient of the first plurality contains differently labelled reagents directed to each different polymorphism; or
(ii) a second plurality of containers containing labelled reagents for detecting the plurality of polymorphisms as defined in (i), each container of the second plurality containing labelled reagents directed to a different polymorphism, wherein the labels are the same or different.

14. A computer-implemented method for detecting donor-derived cell-free nucleic acids in a transplant recipient, the method comprising
instructing assay performance, the assay comprising assaying at least one biological sample derived from the recipient containing cell-free nucleic acids to detect a presence and/or a quantity of one or more markers, wherein the one or more markers consist of one or more donor-informative polymorphisms selected from:
(i) a copy number deletion (CND) comprising from 60 to 100,000 consecutive nucleotides which are present in the genome of the donor but deleted in the genome of the recipient , or
(ii) an allele present in the genome of the donor but not in the genome of the recipient, said allele comprising at least 5 SNPs which are different in the donor and the recipient within a DNA polymorphic locus no longer than 120 nucleotides, in particular wherein said DNA polymorphic locus is within a HLA region;
receiving the presence and/or quantity of the one or more donor-informative polymorphisms in the biological sample;
providing result information about donor-derived cell-free nucleic acids in the transplant recipient.

15. A system for automatically preparing reagents, the system comprising:
- a test plate or rack comprising a plurality of receptacles, each receptacle of the plurality of receptacles configured to accommodate a container; and
- the system configured to receive information relative to a selection of receptacles of the plurality of receptacles wherein the selection of receptacles are configured to accommodate at least a selection of containers, the selection of containers comprising reagents for determining the presence and/or quantity of the one or more donor-informative polymorphisms as defined in claim 1, the reagents in particular comprising primers and labelled probes targeting the one or more donor-informative polymorphisms.

16. An in vitro method for detecting donor-derived cell-free nucleic acids in a transplant recipient, said method comprising assaying at least one biological sample derived from the recipient containing cell-free nucleic acids to detect the presence and/or quantity of two or more donor-informative polymorphisms in a single reaction using labelled reagents directed at said donor-informative polymorphisms, wherein all labelled reagents contain the same detectable label, wherein detecting at least one donor-informative polymorphism is indicative of donor-derived cell-free nucleic acids in the transplant recipient.
